# EUROPEAN PATENT APPLICATION

(11) **EP 4 667 459 A1**
(43) Date of publication of application: **24.12.2025**
(21) Application number: 24756040.2
(22) Date of filing: 03.02.2024
(51) Int. Cl.: C07D 239/42, A01N 43/54, A01P 13/00, C07F 5/02

(54) **BENZENE RING-SUBSTITUTED PYRIMIDINE CARBOXYLIC ACID COMPOUND, AND PREPARATION METHOD THEREFOR, HERBICIDAL COMPOSITION COMPRISING SAME, AND USE THEREOF**

(30) Priority: 17.02.2023 CN 202310127334; 11.04.2023 CN 202310384995; 24.11.2023 CN 202311586870
(71) Applicant: Qingdao KingAgroot Chemical Compound Co., Ltd., Qingdao, Shandong 266000 (CN)
(72) Inventor: LIAN, Lei, Qingdao, Shandong 266000 (CN); PENG, Xuegang, Qingdao, Shandong 266000 (CN); HUA, Rongbao, Qingdao, Shandong 266000 (CN)
(74) Representative: De Vries & Metman
(86) International application number: PCT/CN2024/075642
(87) International publication number: WO 2024/169666

(57) **Abstract**

The present invention belongs to the technical field of pesticides and particularly relates to a benzene ring-substituted pyrimidinecarboxylic acid compound, a preparation method therefor, and a herbicidal composition and an application thereof. The benzene ring-substituted pyrimidinecarboxylic acid compound, as shown in the general formula I: 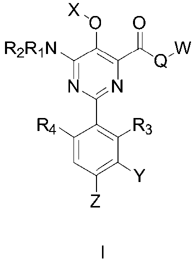 wherein, X represents unsubstituted or halogen-substituted alkyl; Y represents OR₅; Z represents bromine, haloalkyl, or cycloalkyl; Q represents O or S; R₁ and R₂ each independently represent hydrogen; R₃ represents halogen or amino; R₄ represents hydrogen or halogen; R₅ represents unsubstituted or halogen-substituted alkyl; W represents hydrogen or its salt, alkyl, alkenyl, alkynyl, cycloalkyl, etc. The compound has excellent herbicidal activity against gramineous weeds, broad-leaf weeds, etc. and is safe and has high selectivity to crops/vegetables such as maize, wheat, paddy rice, oilseed rape, Chinese cabbage, radish, etc.

## Description

### Technical Field

The present invention belongs to the technical field of pesticides and particularly relates to a benzene ring-substituted pyrimidinecarboxylic acid compound, a preparation method therefor, and a herbicidal composition and an application thereof.

### Background Art

Weed control is one of the most important links in the course of achieving high-efficiency agriculture. Although various herbicides are available in the market, the herbicidal properties against harmful plants and the selectivity to crops of these known compounds are not completely satisfactory. Moreover, due to the continuous expansion of the market, issues such as resistance of weeds, service life and economy of drugs, and people's increasing attention to the environment, scientists are expected to continuously study and further develop new efficient, safe, and economical herbicide varieties with different modes of action.

### Description of the Invention

The present invention provides a benzene ring-substituted pyrimidinecarboxylic acid compound, a preparation method therefor, and a herbicidal composition and an application thereof. The compound has excellent herbicidal activity against gramineous weeds, broad-leaf weeds, etc. and is safe and has high selectivity to crops/vegetables such as maize, wheat, paddy rice, oilseed rape, Chinese cabbage, radish, etc.

The technical scheme adopted in the present invention is as follows:
a benzene ring-substituted pyrimidinecarboxylic acid compound, as shown in the general formula I: 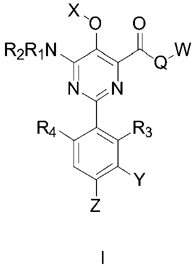
wherein,
X represents unsubstituted or halogen-substituted alkyl;
Y represents OR₅;
Z represents bromine, haloalkyl, or cycloalkyl;
Q represents O or S;
R₁ and R₂ each independently represent hydrogen;
R₃ represents halogen or amino;
R₄ represents hydrogen or halogen;
R₅ represents unsubstituted or halogen-substituted alkyl;
W represents hydrogen or its salt, alkyl, alkenyl, alkynyl, cycloalkyl, cycloalkenyl, heterocyclyl, aryl, 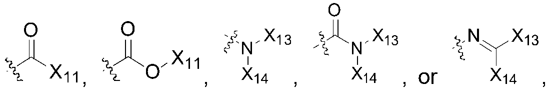 wherein, the "alkyl", "alkenyl" or "alkynyl" is optionally substituted by at least one group selected from halogen, cyano, nitro, cycloalkyl, trialkylsilyl, cycloalkenyl, heterocyclyl, aryl, 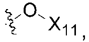 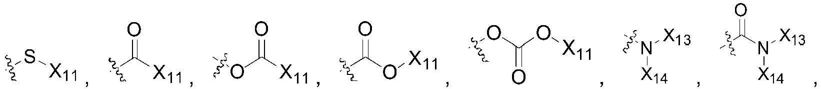 and 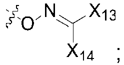 the "cycloalkyl", "cycloalkenyl", "heterocyclyl" or "aryl" is optionally substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, - OR₁₀, -SR₁₀, -(CO)OR₁₀, -(SO₂)R₁₀, -N(R₁₀)₂ and -O-alkylene-(CO)OR₁₀, or two adjacent carbon atoms on the ring form a fused ring with -OCH₂CH₂- or -OCH₂O- that is unsubstituted or substituted by halogen;
X₁₁ each independently represents hydrogen, alkyl, alkenyl, alkynyl, cycloalkyl, cycloalkylalkyl, cycloalkenyl, cycloalkenylalkyl, heterocyclyl, heterocyclylalkyl, aryl, or arylalkyl, wherein, the "alkyl", "alkenyl" or "alkynyl" is optionally substituted by at least one group selected from halogen and alkoxy; the "cycloalkyl", "cycloalkylalkyl", "cycloalkenyl", "cycloalkenylalkyl", "heterocyclyl", "heterocyclylalkyl", "aryl" or "arylalkyl" is optionally substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₀, -SR₁₀, -(CO)OR₁₀, -(SO₂)R₁₀, -N(R₁₀)₂ and -O-alkylene-(CO)OR₁₀, or two adjacent carbon atoms on the ring form a fused ring with -OCH₂CH₂- or -OCH₂O- that is unsubstituted or substituted by halogen;
X₁₃ and X₁₄ each independently represent hydrogen, halogen, cyano, alkoxy, alkoxyalkyl, alkylcarbonyl, alkoxycarbonyl, alkylsulfonyl, alkyl, alkenyl, alkynyl, cycloalkyl, cycloalkylalkyl, cycloalkenyl, cycloalkenylalkyl, aryl, arylalkyl, heterocyclyl or heterocyclylalkyl, or the CX₁₃X₁₄ group together forms an unsubstituted or substituted cyclic structure, or the NX₁₃X₁₄ group together forms an unsubstituted or substituted heterocyclyl with a nitrogen atom at 1-position, wherein, the "alkyl", "alkenyl" or "alkynyl" is optionally substituted by halogen; the "cycloalkyl", "cycloalkylalkyl", "cycloalkenyl", "cycloalkenylalkyl", "aryl", "arylalkyl", "heterocyclyl" or "heterocyclylalkyl" is optionally substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₀, -SR₁₀, -(CO)OR₁₀, -(SO₂)R₁₀, -N(R₁₀)₂ and -O-alkylene-(CO)OR₁₀, or two adjacent carbon atoms on the ring form a fused ring with -OCH₂CH₂- or -OCH₂O- that is unsubstituted or substituted by halogen;
R₁₀ each independently represents hydrogen, alkyl, haloalkyl, phenyl, or phenyl substituted by at least one group selected from halogen, cyano, nitro, alkyl, haloalkyl, alkoxycarbonyl, alkylthio, alkylsulfonyl, alkoxy, and haloalkoxy.

Preferably, X represents unsubstituted or halogen-substituted C1-C8 alkyl;
Z represents bromine, halo C1-C8 alkyl, or C3-C8 cycloalkyl;
R₅ represents unsubstituted or halogen-substituted C1-C8 alkyl;
W represents hydrogen or its metal salt, amine salt, C1-C8 alkyl, C2-C8 alkenyl, C2-C8 alkynyl, C3-C8 cycloalkyl, C3-C8 cycloalkenyl, heterocyclyl, aryl, 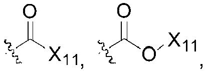 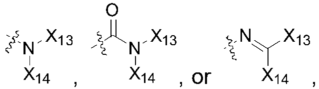 wherein, the "C1-C8 alkyl", "C2-C8 alkenyl" or "C2-C8 alkynyl" is optionally substituted by at least one group selected from halogen, cyano, nitro, C3-C8 cycloalkyl, tri C1-C8 alkylsilyl, C3-C8 cycloalkenyl, heterocyclyl, aryl, 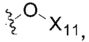 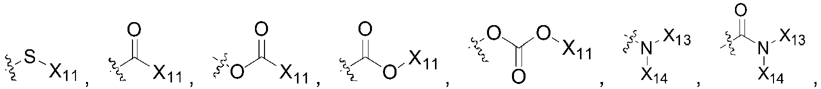 and 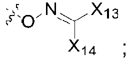 the "C3-C8 cycloalkyl", "C3-C8 cycloalkenyl", "heterocyclyl" or "aryl" is optionally substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₀, -SR₁₀, -(CO)OR₁₀, -(SO₂)R₁₀, -N(R₁₀)₂ and -O-(C1-C8 alkylene)-(CO)OR₁₀, or two adjacent carbon atoms on the ring form a fused ring with -OCH₂CH₂- or -OCH₂O- that is unsubstituted or substituted by halogen;
X₁₁ each independently represents hydrogen, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl, or aryl C1-C8 alkyl, wherein, the "C1-C8 alkyl", "C2-C8 alkenyl" or "C2-C8 alkynyl" is optionally substituted by at least one group selected from halogen and C1-C8 alkoxy; the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" or "aryl C1-C8 alkyl" is optionally substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, - OR₁₀, -SR₁₀, -(CO)OR₁₀, -(SO₂)R₁₀, -N(R₁₀)₂ and -O-(C1-C8 alkylene)-(CO)OR₁₀, or two adjacent carbon atoms on the ring form a fused ring with -OCH₂CH₂- or -OCH₂O- that is unsubstituted or substituted by halogen;
X₁₃ and X₁₄ each independently represent hydrogen, halogen, cyano, C1-C8 alkoxy, C1-C8 alkoxy C1-C8 alkyl, C1-C8 alkylcarbonyl, C1-C8 alkoxycarbonyl, C1-C8 alkylsulfonyl, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, aryl, aryl C1-C8 alkyl, heterocyclyl or heterocyclyl C1-C8 alkyl, or the CX₁₃X₁₄ group together forms a 5-8 membered carbocycle or oxygen-, sulfur- or nitrogen-containing heterocycle, or the NX₁₃X₁₄ group together forms 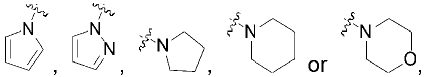 wherein, the "C1-C8 alkyl", "C2-C8 alkenyl" or "C2-C8 alkynyl" is optionally substituted by halogen; the "C3-C8 cycloalkyl", "C3-C8 cycloalkyl C1-C8 alkyl", "C3-C8 cycloalkenyl", "C3-C8 cycloalkenyl C1-C8 alkyl", "aryl", "aryl C1-C8 alkyl", "heterocyclyl" or "heterocyclyl C1-C8 alkyl" is optionally substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₀, -SR₁₀, -(CO)OR₁₀, -(SO₂)R₁₀, -N(R₁₀)₂ and -O-(C1-C8 alkylene)-(CO)OR₁₀, or two adjacent carbon atoms on the ring form a fused ring with -OCH₂CH₂- or -OCH₂O- that is unsubstituted or substituted by halogen; the "5-8 membered carbocycle or oxygen-, sulfur-, or nitrogen-containing heterocycle" is optionally substituted by at least one group selected from C1-C8 alkyl, C1-C8 alkoxycarbonyl and benzyl, or forms a fused ring structure with aryl or heterocyclyl; the 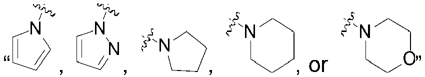 is optionally substituted by at least one group selected from oxo, C1-C8 alkyl, and C1-C8 alkoxycarbonyl;
R₁₀ each independently represents hydrogen, C1-C8 alkyl, halo C1-C8 alkyl, phenyl, or phenyl substituted by at least one group selected from halogen, cyano, nitro, C1-C8 alkyl, halo C1-C8 alkyl, C1-C8 alkoxycarbonyl, C1-C8 alkylthio, C1-C8 alkylsulfonyl, C1-C8 alkoxy, and halo C1-C8 alkoxy.

More preferably, X represents unsubstituted or halogen-substituted C1-C6 alkyl;
Z represents bromine, halo C1-C6 alkyl, or C3-C8 cycloalkyl;
R₅ represents unsubstituted or halogen-substituted C1-C6 alkyl;
W represents hydrogen or its sodium salt, potassium salt, C1-C6 alkyl, C2-C6 alkenyl, C2-C6 alkynyl, C3-C6 cycloalkyl, C3-C6 cycloalkenyl, heterocyclyl, aryl, 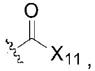 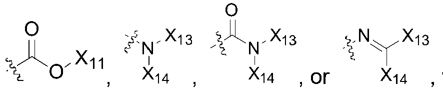 wherein, the "C1-C6 alkyl", "C2-C6 alkenyl" or "C2-C6 alkynyl" is optionally substituted by 1, 2 or 3 groups selected from halogen, cyano, nitro, C3-C6 cycloalkyl, tri C1-C6 alkylsilyl, C3-C6 cycloalkenyl, heterocyclyl, aryl, 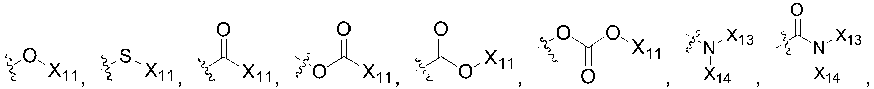 and 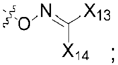 the "C3-C6 cycloalkyl", "C3-C6 cycloalkenyl", "heterocyclyl" or "aryl" is optionally substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₀, -SR₁₀, -(CO)OR₁₀, -(SO₂)R₁₀, -N(R₁₀)₂ and -O-(C1-C6 alkylene)-(CO)OR₁₀, or two adjacent carbon atoms on the ring form a fused ring with -OCH₂CH₂- or -OCH₂O- that is unsubstituted or substituted by halogen;
X₁₁ each independently represents hydrogen, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl, or aryl C1-C6 alkyl, wherein, the "C1-C6 alkyl", "C2-C6 alkenyl" or "C2-C6 alkynyl" is optionally substituted by at least one group selected from halogen and C1-C6 alkoxy; the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" or "aryl C1-C6 alkyl" is optionally substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₀, -SR₁₀, -(CO)OR₁₀, -(SO₂)R₁₀, -N(R₁₀)₂ and -O-(C1-C6 alkylene)-(CO)OR₁₀, or two adjacent carbon atoms on the ring form a fused ring with -OCH₂CH₂- or -OCH₂O- that is unsubstituted or substituted by halogen;
X₁₃ and X₁₄ each independently represent hydrogen, halogen, cyano, C1-C6 alkoxy, C1-C6 alkoxy C1-C6 alkyl, C1-C6 alkylcarbonyl, C1-C6 alkoxycarbonyl, C1-C6 alkylsulfonyl, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, aryl, aryl C1-C6 alkyl, heterocyclyl or heterocyclyl C1-C6 alkyl, or the CX₁₃X₁₄ group together forms a 5-8 membered saturated carbocycle, 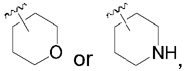 or the NX₁₃X₁₄ group together forms 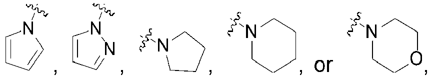 wherein, the "C1-C6 alkyl", "C2-C6 alkenyl" or "C2-C6 alkynyl" is optionally substituted by halogen; the "C3-C6 cycloalkyl", "C3-C6 cycloalkyl C1-C6 alkyl", "C3-C6 cycloalkenyl", "C3-C6 cycloalkenyl C1-C6 alkyl", "aryl", "aryl C1-C6 alkyl", "heterocyclyl" or "heterocyclyl C1-C6 alkyl" is optionally substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₀, -SR₁₀, -(CO)OR₁₀, -(SO₂)R₁₀, -N(R₁₀)₂ and -O-(C1-C6 alkylene)-(CO)OR₁₀, or two adjacent carbon atoms on the ring form a fused ring with -OCH₂CH₂- or -OCH₂O- that is unsubstituted or substituted by halogen; the "5-8 membered saturated carbocycle, 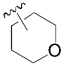 or 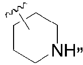 is optionally substituted by 1, 2 or 3 groups selected from C1-C6 alkyl, C1-C6 alkoxycarbonyl and benzyl, or forms a fused ring structure with aryl or heterocyclyl; the 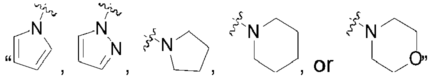 is optionally substituted by 1, 2 or 3 groups selected from oxo, C1-C6 alkyl, and C1-C6 alkoxycarbonyl;
R₁₀ each independently represents hydrogen, C1-C6 alkyl, halo C1-C6 alkyl, phenyl, or phenyl substituted by 1, 2 or 3 groups selected from halogen, cyano, nitro, C1-C6 alkyl, halo C1-C6 alkyl, C1-C6 alkoxycarbonyl, C1-C6 alkylthio, C1-C6 alkylsulfonyl, C1-C6 alkoxy, and halo C1-C6 alkoxy.

Further preferably, X represents methyl, CHF₂, CH₂F, or CF₃;
Z represents bromine, CF₃, or cyclopropyl;
R₃ represents fluorine, chlorine, or amino;
R₄ represents hydrogen or fluorine;
R₅ represents methyl, CHF₂, CH₂F, or CF₃.

The present invention also provides an intermediate compound, as shown in the general formula II: 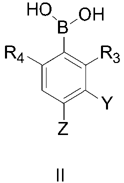 wherein, the definitions of substituents R₃, R₄, Y and Z are as described above.

In the definitions of the compounds represented by the above-mentioned general formulas and all of the following structural formulas, the technical terms used, whether used alone or used in compound words, represent the following substituent groups: an alkyl group which has more than two carbon atoms and may be linear or branched. For example, the alkylene in the compound word "-O-alkylene-(CO)OR₁₀" may be -CH₂-, - CH₂CH₂-, -CH(CH₃)-, -C(CH₃)₂-, etc. An alkyl group is, for example, C1 alkyl: methyl; C2 alkyl: ethyl; C3 alkyl: propyl such as n-propyl or isopropyl; C4 alkyl: butyl such as n-butyl, isobutyl, tert-butyl, or 2-butyl; C5 alkyl: pentyl such as n-pentyl; C6 alkyl: hexyl such as *n-*hexyl, isohexyl, and 1,3-dimethylbutyl. Similarly, alkenyl is, for example, vinyl, allyl, 1-methylprop-2-en-1-yl, 2-methylprop-2-en-1-yl, but-2-en-1-yl, but-3-en-1-yl, 1-methylbut-3-en-1-yl, and 1-methylbut-2-en-1-yl. Alkynyl is, for example, ethynyl, propargyl, but-2-yn-1-yl, but-3-yn-1-yl, and 1-methylbut-3-yn-1-yl. A multiple bond may be at any position of each unsaturated group. Cycloalkyl is a saturated carbocyclic ring system having for example three to six carbon atoms such as cyclopropyl, cyclobutyl, cyclopentyl, or cyclohexyl. Similarly, cycloalkenyl is monocyclic alkenyl having for example three to six carbon ring members such as cyclopropenyl, cyclobutenyl, cyclopentenyl, and cyclohexenyl, wherein a double bond can be at any position. A halogen is fluorine, chlorine, bromine, or iodine.

Unless otherwise specified, the "aryl" of the present invention includes, but is not limited to, phenyl, naphthyl, 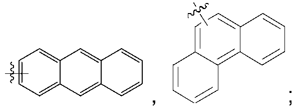 the "heterocyclyl" not only includes, but is not limited to, saturated or unsaturated non-aromatic cyclic groups 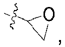 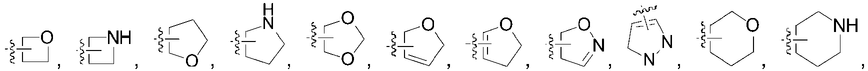 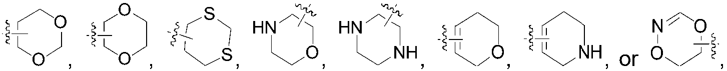 etc., but also includes, but is not limited to, heteroaryl, that is an aromatic cyclic group having for example 3 to 6 ring atoms and also optionally fused with a benzo ring. One to four (for example, 1, 2, 3, or 4) heteroatoms of the ring atoms are selected from oxygen, nitrogen, and sulfur, for example

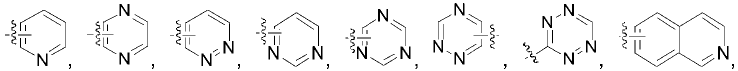

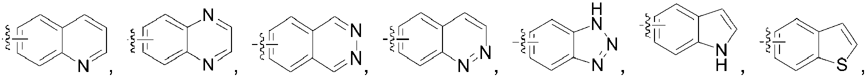

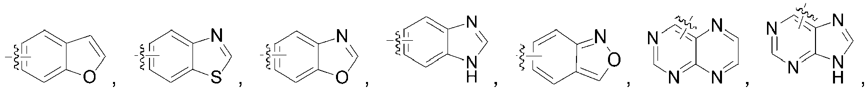

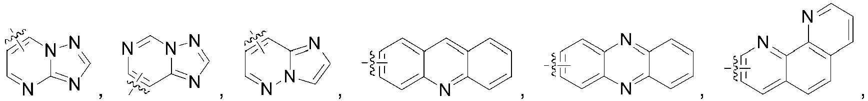

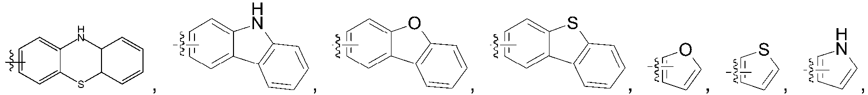

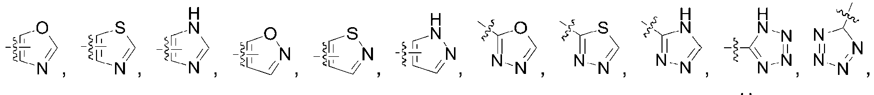

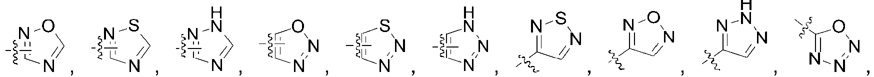

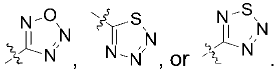

If one group is substituted by a group, which should be understood to mean that the group is substituted by one or more same or different groups selected from the mentioned groups. In addition, the same or different substitution characters contained in the same or different substituent groups are independently selected, which may be the same or different. This is also applicable to a ring system formed by different atoms and units. Meanwhile, the scope of the claims will exclude those compounds chemically unstable under standard conditions which are known to a person skilled in the art.

In addition, unless specifically defined, the term "substituted by at least one group" herein refers to being substituted by for example 1, 2, 3, 4, or 5 groups; a group (including heterocyclyl, aryl, etc.) without being specified a linking site may be linked at any site, including a site connected to C or N; if it is substituted, the substituent group may also substitute at any site as long as the valence bond theory is complied with. For example, the heteroaryl 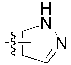 substituted by 1 methyl group may represent 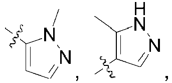 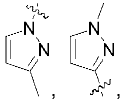 etc.

According to the nature of substituent groups and the manner in which they are connected, the compounds of the general formula I and derivatives thereof may exist as stereoisomers. For example, if one or more asymmetric carbon atoms are present, enantiomers and diastereoisomers may occur. Stereoisomers may be obtained by conventional separation methods, for example by chromatographic separation, from mixtures produced in preparation. Stereoisomers may also be selectively prepared by employing stereoselective reactions and using optically active starting materials and/or auxiliaries. The present invention further relates to all stereoisomers which are included in the general formula I but not specifically defined, and mixtures thereof.

The preparation method for the benzene ring-substituted pyrimidinecarboxylic acid compounds comprises the following steps:
subjecting the compound shown in the general formula II and the compound shown in the general formula III to reaction to produce the compound as shown in the general formula I, and the reaction equation is as follows: 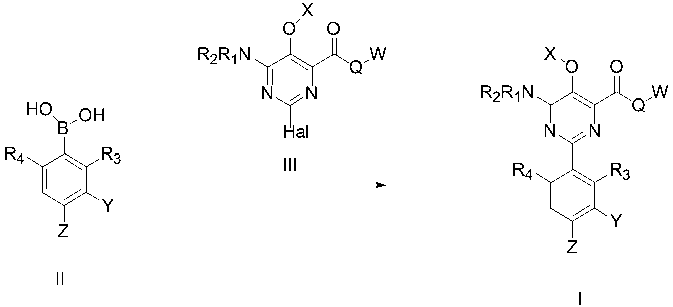
wherein, Hal represents halogen, preferably Cl; the definitions of R₁, R₂, R₃, R₄, X, Y, Z, Q and W are as described before.

Preferably, the reaction is carried out in the presence of a catalyst, a base, and a solvent.

In one specific embodiment, the catalyst is a palladium catalyst [e.g., Pd(dppf)Cl₂·CH₂Cl₂, Pd(dppf)Cl₂, Pd(PPh₃)₄, methanesulfonato(2-dicyclohexylphosphino-2',6'-dimethoxybiphenyl)(2'-methylamino-1,1'-biphenyl-2-yl)palladium(II) (CAS No.: 1599466-87-1), or methanesulfonato(tri-tert-butylphosphino)(2'-methylamino-1,1'-biphenyl-2-yl)palladium(II) (CAS No.: 1621274-11-0), etc.] or nickel catalyst.

In another specific embodiment, the base is selected from at least one of inorganic bases (e.g., K₂CO₃, Na₂CO₃, Cs₂CO₃, NaHCO₃, KHCO₃, KF, CsF, K₃PO₄, K₂HPO₄, NaOH, KOH, etc.) and organic bases (e.g., pyrazole, triethylamine, DIEA, potassium trimethylsilanolate, AcOK, AcONa, MeONa, EtONa, t-BuONa, etc.).

In another specific embodiment, the solvent is organic solvent/water, the volume ratio of which is preferably 20/1-1/1, and the organic solvent is preferably dioxane, toluene, DMF, DMSO, DMA, methanol, ethanol, isopropanol, n-butanol, acetonitrile, dichloroethane, dichloromethane, or ethyl acetate.

The compounds as shown in the general formula I can be prepared by referring to the methods in WO2009029735 and the like.

A herbicidal composition comprises a herbicidally effective amount of at least one of the benzene ring-substituted pyrimidinecarboxylic acid compounds, preferably, also comprising a formulation auxiliary.

A method for controlling weeds comprises applying a herbicidally effective amount of at least one of the benzene ring-substituted pyrimidinecarboxylic acid compounds or the herbicidal composition to a plant or a weedy area.

At least one of the benzene ring-substituted pyrimidinecarboxylic acid compounds or the herbicidal composition has use in controlling weeds; preferably, the benzene ring-substituted pyrimidinecarboxylic acid compound is used for preventing and controlling weeds among useful crops, and the useful crops are transgenic crops or crops treated by genome editing techniques.

The compounds of Formula I of the present invention have outstanding herbicidal activity against a broad spectrum of monocotyledonous and dicotyledonous harmful plants of economic importance. The active substances of the present invention also act efficiently on perennial weeds which grow from root stocks, rhizomes, or other perennial organs and are difficult to control. In this context, it is generally immaterial whether the substances are applied before sowing, before emergence, or after emergence. Representative examples of monocotyledonous and dicotyledonous weed florae which can be controlled by the compounds of the present invention may be specifically mentioned, without limiting to certain species. Examples of weed species on which the active substances act efficiently include the monocotyledons such as annual *Avena, Lolium, Alopecurus, Phalaris, Echinochloa, Digitaria, Setaria* and *Cyperus,* as well as perennial *Agropyron, Cynodon, Imperata* and *Sorghum,* and perennial *Cyperus.*

In the case of dicotyledonous weed species, the spectrum of action extends to species such as annual *Galium, Viola, Veronica, Lamium, Stellaria, Amaranthus, Sinapis, Ipomoea, Sida, Matricaria* and *Abutilon,* as well as perennial weeds *Convolvulus, Cirsium, Rumex,* and *Artemisia.* The active substances of the present invention perform outstanding control of harmful plants such as *Echinochloa, Sagittaria, Alisma, Eleocharis, Scirpus* and *Cyperus* under the specific condition of paddy rice growing. If the compounds of the present invention are applied to soil surface prior to germination, the weed seedlings are either prevented completely from emerging, or the weeds stop growing when reaching the cotyledon stage and eventually died completely after three to four weeks. In particular, the compounds of the present invention exhibit excellent activity against *Apera spica venti, Matsumurella chinense, Fallopia convolvulus, Stellaria media, Veronica hederifolia, Veronica persica, Viola tricolor, Amaranthus, Galium,* and *Kochia.*

Although the compounds of the present invention have excellent herbicidal activity against monocotyledonous and dicotyledonous weeds, there is no damage at all to crop plants of economic importance such as wheat, barley, rye, rice, maize, sugarbeet, cotton and soybean, or the damage is negligible. In particular, they have excellent compatibility with cereals such as wheat, barley, and maize, in particular wheat. Therefore, the compounds of the present invention are highly suitable for selectively controlling undesired plants in plantings for agricultural or ornamental use.

Owing to their herbicidal properties, these active substances may be employed for controlling harmful plants in plantings of genetically engineered plants that are known or to be introduced. Transgenic plants usually have advantageous traits, for example, resistance to certain pesticides, in particular to certain herbicides; resistance to plant diseases or pathogenic microorganisms of plant diseases such as certain insects or microorganisms including fungi, bacteria, or viruses. Other particular traits relate to the following conditions of the product, for example, quantity, quality, storage stability, composition, and special ingredients. Thus, it is known that the obtained transgenic plant product has an increased starch content, or modified starch quality, or a different fatty acid composition.

The compounds of Formula I of the present invention or salts thereof are preferably used in plantings of transgenic crops and ornamental plants of economic importance, for example, cereals such as wheat, barley, rye, oats, millet, rice, manioc, and maize; or in plantings of vegetable plants such as sugarbeet, cotton, soybean, rapeseed, potato, tomato, pea and the like. The compounds of Formula I are preferably used as herbicides in plantings of useful plants which are resistant, or have been made resistant by genetic engineering, against the toxic effects of the herbicides.

Conventional ways for breeding plants which have modified traits compared to known plants include, for example, conventional breeding methods and breeding of mutant strains. In other words, novel plants having improved traits may be generated with the aid of genetic engineering methods (see, e.g., EP-0221044 A, EP-0131624 A). For example, several methods have been described:
- changing crop plants using genetic engineering to modify the starch synthesis in plants (e.g., WO 92/11376, WO 92/14827, WO 91/19806);
- transgenic crop plants resistant to certain herbicides, including glufosinate herbicides (e.g., EP-0242236 A, EP-0242246 A), or glyphosate herbicides (WO 92/00377), or sulfonylurea herbicides (EP-0257993 A, US-5013659 A);
- transgenic crop plants, for example cotton, able to produce *Bacillus thuringiensis* toxins *(Bt* toxins) which impart resistance to certain pests that invade the plants (EP-0142924 A, EP-0193259 A);
- transgenic crop plants having a modified fatty acid composition (WO 91/13972).

Numerous molecular biology techniques which allow preparation of transgenic plants having modified traits are known (see, e.g., Sambrook et al., 1989, Molecular Cloning, A Laboratory Manual, 2nd edition, Cold Spring Harbor Laboratory Press, Cold Spring Harbor, N.Y.; or Winnacker, "Gene und Klone" [Genes and Clones], VCH Weinheim, 2nd edition, 1996; or Christou, "Trends in Plant Science", 1 (1996) 423-431). In order to carry out genetic engineering manipulations, it is possible to introduce nucleic acid molecules into plasmids, allowing mutagenesis or changes in sequences to occur by recombination of DNA sequences. Using the above-mentioned standard processes, it is possible to, for example, substitute bases, remove partial sequences, or add natural or synthetic sequences. To link DNA fragments with each other, it is possible to attach adaptors or linkers to the fragments.

A plant cell having a gene product of reduced activity may be prepared by the following methods, for example, by expressing at least one appropriate antisense-RNA and one sense-RNA to achieve a cosuppression effect, or by expressing at least one appropriately constructed ribozyme which specifically cleaves the transcripts of the above-mentioned gene product.

To this end, it is possible to employ a DNA molecule which comprises all the coding sequences of the gene product including any flanking sequence that may be present, and a DNA molecule which comprises only parts of the coding sequences that must be long enough to cause an antisense effect in the cells. It is also possible to use sequences which have a high degree of homology but are not entirely identical to the coding sequences of the gene product.

When nucleic acid molecules are expressed in a plant, the synthesized protein may be localized in any desired compartment of the plant cell. However, to achieve localization in a certain compartment, it is possible, for example, to link the coding region with the DNA sequences to ensure localization in the certain compartment. Such sequences are known to a person skilled in the art (see, e.g., Braun et al., EMBO J., 11 (1992), 3219-3227; Wolter et al., Proc. Natl. Acad. Sci. USA, 85 (1988), 846-850; Sonnewald et al., Plant J., 1 (1991), 95-106).

Transgenic plant cells can be recombined onto a whole plant using known techniques. A transgenic plant may be of any desired plant species, i.e., a monocotyledonous and dicotyledonous plant. In this manner, it is possible to obtain transgenic plants having modified traits by overexpression, suppression, or inhibition of homologous (= natural) genes or gene sequences, or by expression of heterologous (= foreign) genes or gene sequences.

When using the active substances of the present invention in transgenic crops, in addition to the inhibitory effect against harmful plants which can be observed in other crops, there are frequently special effects on the corresponding transgenic crops, for example, improving or broadening the spectrum of weeds which can be controlled, modifying the application amount during application, excellent combination of the drug resistance of preferred transgenic crops and herbicide performance, as well as effects on the growth and the yield of transgenic crop plants. The present invention therefore also provides use of the compounds as herbicides for controlling harmful plants among transgenic crop plants.

In addition, the compounds of the present invention are able to significantly regulate the growth of crop plants. These compounds are employed for the targeted control of plant constituents and for facilitating harvesting by engaging in plant metabolism in a regulating manner, for example by provoking desiccation and stunted growth. Furthermore, they are also suitable for regulating and inhibiting undesirable plant growth without destroying the growth of crop plants. Inhibition of plant growth plays a key role in many monocotyledonous and dicotyledonous crops because it can reduce or completely prevent lodging hereby.

The compounds of the present invention may be applied in customary formulations in the form of wettable powders, emulsifiable concentrates, sprayable solutions, dusts, or granules. The present invention therefore also provides herbicidal compositions comprising the compounds of Formula I. The compounds of Formula I may be formulated in numerous ways depending on the prevailing physical parameters in biology and/or chemistry. Examples of suitable formulation options are: wettable powders (WP), water-soluble powders (SP), water-soluble concentrates, emulsifiable concentrates (EC), emulsions such as oil-in-water and water-in-oil emulsions (EW), sprayable solutions, suspension concentrates (SC), oil dispersions (OD), oil- or water-based dispersions, oil-miscible solutions, dust powders (DP), capsule suspensions (CS), seed-dressing compositions, granules for broadcasting and soil application, spray granules, coating granules and adsorption granules, water-dispersible granules (WG), water-soluble granules (SG), ULV (Ultra-low volume) formulations, microcapsules, and waxes. These individual formulation types are known and described in the following literature, for example, Winnacker-Küchler, "Chemische Technologie" [Chemical Technology], Volume 7, C. Hauser Verlag Munich, 4th edition, 1986; Wade van Valkenburg, "Pesticide Formulations", Marcel Dekker, N.Y., 1973; K. Martens, "Spray Drying" Handbook, 3rd edition, 1979, G. Goodwin Ltd. London.

Necessary formulation auxiliaries such as inert materials, surfactants, solvents and other additives are likewise known and described in the following documents, for example, Watkins, "Handbook of Insecticide Dust Diluents and Carriers", 2nd edition, Dorland Books, Caldwell N.J.; H. v. Olphen, "An Introduction to Clay Colloid Chemistry", 2nd edition, J. Wiley & Sons, N.Y.; C. Marsden, "Solvents Guide", 2nd edition, Interscience, N.Y. 1963; "McCutcheon's Detergents and Emulsifiers Annual", MC Publ. Corp., Ridgewood N.J.; Sisley and Wood, "Encyclopedia of Surface Active Agents", Chem. Publ. Co. Inc., N.Y., 1964; Schönfeldt, "Grenzflüchenaktive Äthylenoxidaddkte" [Surface-active ethylene oxide adducts], Wiss. Verlagagesell., Stuttgart, 1976; Winnacker-Küchler, "Chemische Technologie" [Chemical Technology], Volume 7, C. Hauser Verlag Munich, 4th edition, 1986.

Wettable powders are uniformly dispersible in water which contain, in addition to the active substance, a diluent or inert substance, ionic and nonionic surfactant (wetting agent, dispersant), for example polyethoxylated alkyl phenol, polyethoxylated fatty alcohol, polyethoxylated fatty amine, fatty alcohol polyglycol ether sulfate, alkane sulfonate, alkylphenyl sulfonate, sodium lignosulfonate, sodium 2,2'-dinaphthylmethane-6,6'-disulfonats, sodium dibutylnaphthalenesulfonate, or sodium methyl oleoyl taurate. To prepare the wettable powders, the herbicidally active substance is finely ground, for example, in a customary apparatus such as hammer mill, fan mill and air-jet mill, and mixed with formulation auxiliaries simultaneously or sequentially.

Emulsifiable concentrates are prepared by dissolving the active substance in an organic solvent, for example butanol, cyclohexanone, dimethylformamide, xylene, or aromatic compounds with relatively high boiling point, or hydrocarbons or mixtures of the solvents, with the addition of one or more ionic and/or nonionic surfactants (emulsifiers). Examples of emulsifiers which can be used are calcium alkylarylsulfonates such as calcium dodecylbenzene sulfonate, or nonionic emulsifiers such as fatty acid polyglycol esters, alkylaryl polyglycol ethers, fatty alcohol polyglycol ethers, propylene oxide-ethylene oxide condensation products, alkyl polyethers, sorbitan esters such as sorbitan fatty acid esters, or polyoxyethylene sorbitan esters such as polyoxyethylene sorbitan fatty acid esters.

Dusts are obtained by grinding the active substance with finely-divided solid substances, for example talc, natural clays such as kaolin, bentonite and pyrophyllite, or diatomaceous earth. Water- or oil-based suspensions may be prepared by the following methods, for example, by wet milling using a commercially customary bead mill, with or without the addition of the surfactants mentioned above in the case of another formulation type.

Emulsions such as oil-in-water emulsions (EW) may be prepared by means of stirrers, colloid mills and/or static mixers using aqueous organic solvents, and, if desired, the surfactants as mentioned above in the case of another formulation type may be added.

Granules may be prepared by the following methods that either spraying the active substance onto the adsorptive and granulating with inert materials, or concentrating the active substance onto the surface of carriers such as sand and kaolinite and granulating inert materials by means of adhesive binders such polyvinyl alcohol, sodium polyacrylate, or mineral oils. Suitable active substances may also be granulated in the manner which is customary for the preparation of fertilizer granules. If desired, fertilizers may be mixed in. Water-dispersible granules are prepared by customary methods such as spray-drying, fluidized-bed granulation, disk granulation, or mixing using high-speed mixers and extrusion without solid inert materials.

For the preparation methods of granules using disk, fluidized-bed, extruder and spray, see the following processes in, for example, "Spray-Drying Handbook" 3rd edition, 1979, G. Goodwin Ltd., London; J. E. Browning, "Agglomeration", Chemical and Engineering, 1967, pages 147 ff.; "Perry's Chemical Engineer's Handbook", 5th edition, McGraw-Hill, New York 1973, pp. 8-57. For further details on the formulation of crop protection products, see, for example G. C. Klingman, "Weed Control as a Science", John Wiley and Sons Inc., New York, 1961, pages 81-96 and J. D. Freyer, S. A. Evans, "Weed Control Handbook", 5th edition, Blackwell Scientific Publications, Oxford, 1968, pages 101-103.

An agrochemical formulation generally contains from 0.1 to 99% by weight, in particular from 0.1 to 95%, of the active substance of Formula I. In a wettable powder, the concentration of the active substance is, for example, from about 10 to 99% by weight, and the remainder to 100% by weight consists of customary formulation constituents. In an emulsifiable concentrate, the concentration of the active substance may be from about 1 to 90%, preferably from 5 to 80%, by weight. A dust formulation contains from 1 to 30% by weight of the active substance, preferably from 5 to 20% by weight of the active substance in usual cases, while a sprayable solution contains from about 0.05 to 80%, preferably from 2 to 50%, by weight of the active substance. In the case of water-dispersible granules, the content of the active substance mainly depends on whether the active substance is liquid or solid and on auxiliaries, fillers, etc. that are used during granulation. In water-dispersible granules, the content of the active substance, for example, is between 1 and 95% by weight, preferably between 10 and 80% by weight.

In addition, the formulations of the active substances may include tackifiers, wetting agents, dispersants, emulsifiers, penetrants, preservatives, antifreeze agents, solvents, fillers, carriers, colorants, defoamers, evaporation suppressors as well as pH and viscosity modifiers which are usually customary in all cases.

Based on these formulations, it is also possible to produce mixtures with other pesticidally active substances, for example insecticides, acaricides, herbicides and fungicides, and also with safeners, fertilizers and/or plant growth regulators, in a manner of pre-mix or tank mix.

In mixed formulations or tank mix formulations, suitable active substances which can be mixed with the active substances of the present invention are the known substances described, for example in World Herbicide New Product Technology Handbook, China Agricultural Science and Farming Techniques Press, 2010. 9 and in the literature cited herein. For example, the herbicidally active substances mentioned below may be mixed with the compounds of Formula I (noted that the compounds are either named by the "common name" in accordance with the International Organization for Standardization (ISO) or by the chemical names, accompanied with a customary code number if appropriate): acetochlor, butachlor, alachlor, propisochlor, metolachlor, S-metolachlor, pretilachlor, propachlor, ethachlor, napropamide, R-left handed napropamide, propanil, mefenacet, diphenamid, diflufenican, ethaprochlor, beflubutamid, bromobutide, dimethenamid, dimethenamid-P, etobenzanid, flufenacet, thenylchlor, metazachlor, isoxaben, flamprop-M-methyl, flamprop-M-propyl, allidochlor, pethoxamid, chloranocryl, cypromid, mefluidide, monalide, delachlor, prynachlor, terbuchlor, xylachlor, dimethachlor, cisanilide, trimexachlor, clomeprop, propyzamide, pentanochlor, carbetamide, benzoylprop-ethyl, cyprazole, butenachlor, tebutam, benzipram, quinonamid, dichlofluanid, naproanilide, diethatyl-ethyl, naptalam, flufenacet, benzadox, chlorthiamid, chlorophthalimide, isocarbamide, picolinafen, atrazine, simazine, prometryn, cyanatryn, simetryn, ametryn, propazine, dipropetryn, SSH-108, terbutryn, terbuthylazine, triaziflam, cyprazine, proglinazine, trietazine, prometon, simetone, aziprotryne, desmetryn, dimethametryn, procyazine, mesoprazine, sebuthylazine, secbumeton, terbumeton, methoprotryne, cyanatryn, ipazine, chlorazine, atraton, pendimethalin, eglinazine, cyanuric acid, indaziflam, chlorsulfuron, metsulfuron-methyl, bensulfuron-methyl, chlorimuron-ethyl, tribenuron-methyl, thifensulfuron-methyl, pyrazosulfuron-ethyl, mesosulfuron, iodosulfuron-methyl sodium, foramsulfuron, cinosulfuron, triasulfuron, sulfometuron methyl, nicosulfuron, ethametsulfuron-methyl, amidosulfuron, ethoxysulfuron, cyclosulfamuron, rimsulfuron, azimsulfuron, flazasulfuron, monosulfuron, monosulfuron-ester, flucarbazone-sodium, flupyrsulfuron-methyl, halosulfuron-methyl, oxasulfuron, imazosulfuron, primisulfuron, propoxycarbazone, prosulfuron, sulfosulfuron, trifloxysulfuron, triflusulfuron-methyl, tritosulfuron, sodium metsulfuron methyl, flucetosulfuron, HNPC-C9908, orthosulfamuron, propyrisulfuron, metazosulfuron, acifluorfen, fomesafen, lactofen, fluoroglycofen, oxyfluorfen,chlornitrofen, aclonifen, ethoxyfen-ethyl, bifenox, nitrofluorfen, chlomethoxyfen, fluorodifen, fluoronitrofen, furyloxyfen, nitrofen, TOPE, DMNP, PPG1013, AKH-7088, halosafen, chlortoluron, isoproturon, linuron, diuron, dymron, fluometuron, benzthiazuron, methabenzthiazuron, cumyluron, ethidimuron, isouron, tebuthiuron, buturon, chlorbromuron, methyldymron, phenobenzuron, SK-85, metobromuron, metoxuron, afesin, monuron, siduron, fenuron, fluothiuron, neburon, chloroxuron, noruron, isonoruron, 3-cyclooctyl-1, thiazfluron, tebuthiuron, difenoxuron, parafluron, methylamine tribunil, karbutilate, trimeturon, dimefuron, monisouron, anisuron, methiuron, chloreturon, tetrafluron, phenmedipham, phenmedipham-ethyl, desmedipham, asulam, terbucarb, barban, propham, chlorpropham, rowmate, swep, chlorbufam, carboxazole, chlorprocarb, fenasulam, BCPC, CPPC, carbasulam, butylate, benthiocarb, vemolate, molinate, triallate, dimepiperate, esprocarb, pyributicarb, cycloate, avadex, EPTC, ethiolate, orbencarb, pebulate, prosulfocarb, tiocarbazil, CDEC, dimexano, isopolinate, methiobencarb, 2,4-D butyl ester, MCPA-Na, 2,4-D isooctyl ester, MCPA isooctyl ester, 2,4-D sodium salt, 2,4-D dimethyla mine salt, MCPA-thioethyl, MCPA, 2,4-D propionic acid, high 2,4-D propionic acid salt, 2,4-D butyric acid, MCPA propionic acid, MCPA propionic acid salt, MCPA butyric acid, 2,4,5-D, 2,4,5-D propionic acid, 2,4,5-D butyric acid, MCPA amine salt, dicamba, erbon, chlorfenac, saison, TBA, chloramben, methoxy-TBA, diclofop-methyl, fluazifop-butyl, fluazifop-P-butyl, haloxyfop-methyl, haloxyfop-P, quizalofop-ethyl, quizalofop-P-ethyl, fenoxaprop-ethy, fenoxaprop-P-ethyl, propaquizafop, cyhalofop-butyl, metamifop, clodinafop-propargyl, fenthiapropethyl, chloroazifop-propynyl, poppenate-methyl, trifopsime, isoxapyrifop, paraquat, diquat, oryzalin, ethalfluralin, isopropalin, nitralin, profluralin, prodinamine, benfluralin, fluchloraline, dinitramina, dipropalin, chlomidine, methalpropalin, dinoprop, glyphosate, anilofos, glufosinate ammonium, amiprophos-methyl, sulphosate, piperophos, bialaphos-sodium, bensulide, butamifos, phocarb, 2,4-DEP, H-9201, zytron, imazapyr, imazethapyr, imazaquin, imazamox, imazamox ammonium salt, imazapic, imazamethabenz-methyl, fluroxypyr, fluroxypyr isooctyl ester, clopyralid, picloram, trichlopyr, dithiopyr, haloxydine, 3,5,6-trichloro-2-pyridinol, thiazopyr, fluridone, aminopyralid, diflufenzopyr, triclopyr-butotyl, Clio-dinate, sethoxydim, clethodim, cycloxydim, alloxydim, clefoxydim, butroxydim, tralkoxydim, tepraloxydim, buthidazole, metribuzin, hexazinone, metamitron, ethiozin, ametridione, amibuzin, bromoxynil, bromoxynil octanoate, ioxynil octanoate, ioxynil, dichlobenil, diphenatrile, pyraclonil, chloroxynil, iodobonil, flumetsulam, florasulam, penoxsulam, metosulam, cloransulam-methyl, diclosulam, pyroxsulam, benfuresate, bispyribac-sodium, pyribenzoxim, pyriftalid, pyriminobac-methyl, pyrithiobac-sodium, benzobicylon, mesotrione, sulcotrione, tembotrione, tefuryltrione, bicyclopyrone, ketodpiradox, isoxaflutole, clomazone, fenoxasulfone, methiozolin, fluazolate, pyraflufen-ethyl, pyrazolynate, difenzoquat, pyrazoxyfen, benzofenap, nipyraclofen, pyrasulfotole, topramezone, pyroxasulfone, cafenstrole, flupoxam, aminotriazole, amicarbazone, azafenidin, carfentrazone-ethyl, sulfentrazone, bencarbazone, benzfendizone, butafenacil, bromacil, isocil, lenacil, terbacil, flupropacil, cinidon-ethyl, flumiclorac-pentyl, flumioxazin, S-23121, MK-129, flumezin, pentachlorophenol, dinoseb, dinoterb, dinoterb acetate, dinosam, DNOC, chloronitrophene, medinoterb acetate, dinofenate, oxadiargyl, oxadiazon, pentoxazone, Flufenacet, fluthiacet-methyl, fentrazamide, flufenpyr-ethyl, pyrazon, brompyrazon, metflurazon, kusakira, dimidazon, oxapyrazon, norflurazon, pyridafol, quinclorac, quinmerac, bentazone, pyridate, oxaziclomefone, benazolin-ethyl, clomazone, cinmethylin, ZJ0702, pyribambenz-propyl, indanofan, sodium chlorate, dalapon, trichloroacetic acid, monochloroacetic acid, hexachloroacetone, flupropanate, cyperquat, bromofenoxim, epronaz, methazole, flurtamone, benfuresate, ethofumesate, tioclorim, chlorthal, fluorochloridone, tavron, acrolein, bentranil, tridiphane, chlorfenpropmethyl, thidiarizonaimin, phenisopham, busoxinone, methoxyphenone, saflufenacil, clacyfos, chloropon, alorac, diethamquat, etnipromid, iprymidam, ipfencarbazone, thiencarbazone-methyl, pyrimisulfan, chlorflurazole, tripropindan, sulglycapin, prosulfalin, cambendichlor, aminocyclopyrachlor, rodethanil, benoxacor, fenclorim, flurazole, fenchlorazole-ethyl, cloquintocet-mexyl, oxabetrinil, MG/91, cyometrinil, DKA-24, mefenpyr-diethyl, furilazole, fluxofenim, isoxadifen-ethyl, dichlormid, halauxifen-methyl, DOW848, UBH-509, D489, LS 82-556, KPP-300, NC-324, NC-330, KH-218, DPX-N8189, SC-0744, DOWCO535, DK-8910, V-53482, PP-600, MBH-001, KIH-9201, ET751, KIH-6127 and KIH-2023.

In a specific embodiment, the active substance is selected from one or more of the following compounds:
(1) HPPD inhibitors: 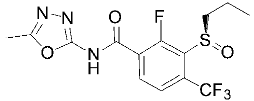 (CAS No.: 2421252-30-2), bipyrazone (CAS No.: 1622908-18-2), fenpyrazone (CAS No.: 1992017-55-6), cypyrafluone (CAS No.: 1855929-45-1), tripyrasulfone (CAS No.: 1911613-97-2);
(2) ALS inhibitors: florasulam (CAS No.: 145701-23-1);
(3) Synthetic hormones: halauxifen-methyl (CAS: CAS No.: 943831-98-9), florpyrauxifen-benzyl (CAS: 1390661-72-9), fluroxypyr-meptyl (CAS No.: 81406-37-3), benazolin-ethyl (CAS No.: 25059-80-7), clopyralid (CAS No.: 1702-17-6), aminopyralid (CAS No.: 150114-71-9), picloram (CAS No.: 1918-02-1), MCPA-isooctyl (CAS No.: 26544-20-7);
(4) PSII inhibitors: propanil (CAS No.: 709-98-8), bentazone (CAS No.: 25057-89-0);
(5) DOXP inhibitors: 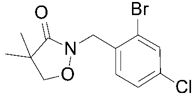 (CAS No.: 2766607-82-1);
(6) FAT inhibitors:

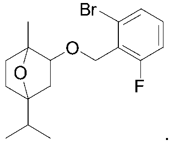

In another specific embodiment, the weight ratio of the benzene ring-substituted pyrimidinecarboxylic acid compound as shown in Formula I and the aforementioned active substance in the composition is 1:500-20:1, 1:400-10:1, 1:300-5:1, 1:200-2:1, 1:100-2:1, 1:50-2:1, 1:30-2:1, 1:20-2:1, 1:10-2:1, or 1:5-1:1.

For use, the commercially available formulations are, if needed, diluted in customary manners, for example using water in the cases of wettable powders, emulsifiable concentrates, dispersions, and water-dispersible granules. For products in the form of dusts, granules for soil application, or solutions for broadcasting and spray, usually no further dilution with inert materials is required prior to use. The required application amount of the compounds of Formula I varies with the external conditions such as temperature, humidity, the nature of the used herbicide and the like. It may have a large variation range, for example, between 0.001 and 1.0 kg a.i./ha, or more active substances, but preferably between 0.005 and 750 g a.i./ha, in particular between 0.005 and 250 g a.i./ha.

### Detailed Embodiments of the Invention

The following examples are used to illustrate the present invention and should not be construed as limiting the present invention in any way. The scope of rights claimed by the present invention is described in the claims.

In view of the economy and diversity of the compounds, some compounds were preferably synthesized. Among the many synthesized compounds, selected ones are listed in Table 1 below. The specific compound structures and corresponding compound information are as set forth in Tables 1-2. The compounds in Table 1 are only to better illustrate the present invention but do not limit the present invention. For a person skilled in the art, it should not be understood that the scope of the above-mentioned subject matters of the present invention is limited to the following compounds.

**Table 1 Compound structure**

| 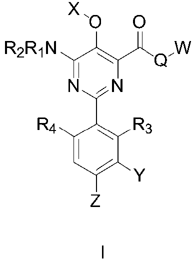 | | | | | | | | | |
|---|---|---|---|---|---|---|---|---|---|
| **No.** | **R₁** | **R₂** | **R₃** | **R₄** | **X** | **Y** | **Z** | **Q** | **W** |
| 1 | H | H | F | H | CH₃ | OCH₃ | CF₃ | O | H |
| 2 | H | H | Cl | H | CH₃ | OCH₃ | CF₃ | O | CH₃ |
| 3 | H | H | F | H | CH₃ | OCH₃ | CF₃ | O | CH₃ |
| 4 | H | H | F | H | CH₃ | OCH₃ | CHF₂ | O | CH₃ |
| 5 | H | H | F | H | CH₃ | OCH₃ | CH₂F | O | CH₃ |
| 6 | H | H | F | H | CH₃ | OCH₃ | CF₂CF₃ | O | CH₃ |
| 7 | H | H | NH₂ | H | CH₃ | OCH₃ | CF₃ | O | CH₃ |
| 8 | H | H | F | F | CH₃ | OCH₃ | CF₃ | O | CH₃ |
| 9 | H | H | F | Cl | CH₃ | OCH₃ | CF₃ | O | CH₃ |
| 10 | H | H | F | Br | CH₃ | OCH₃ | CF₃ | O | CH₃ |
| 11 | H | H | F | H | CH₂CH₃ | OCH₃ | CF₃ | O | CH₃ |
| 12 | H | H | F | H | 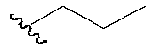 | OCH₃ | CF₃ | O | CH₃ |
| 13 | H | H | F | H | 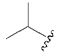 | OCH₃ | CF₃ | O | CH₃ |
| 14 | H | H | F | H | CF₃ | OCH₃ | CF₃ | O | CH₃ |
| 15 | H | H | F | H | CHF₂ | OCH₃ | CF₃ | O | CH₃ |
| 16 | H | H | F | H | CH₂F | OCH₃ | CF₃ | O | CH₃ |
| 17 | H | H | F | H | 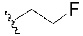 | OCH₃ | CF₃ | O | CH₃ |
| 18 | H | H | F | H | 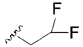 | OCH₃ | CF₃ | O | CH₃ |
| 19 | H | H | F | H | 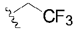 | OCH₃ | CF₃ | O | CH₃ |
| 20 | H | H | F | H | CH₃ | OCH₂CH₃ | CF₃ | O | CH₃ |
| 21 | H | H | F | H | CH₃ | OCH₃ | CF₃ | O | 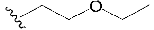 |
| 22 | H | H | F | H | CH₃ | OCH₃ | CF₃ | O | 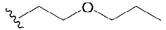 |
| 23 | H | H | F | H | CH₃ | OCH₃ | CF₃ | O | 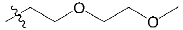 |
| 24 | H | H | F | H | CH₃ | OCH₃ | CF₃ | O | CH₂CH₃ |
| 25 | H | H | F | H | CH₃ | OCH₃ | CF₃ | O | 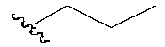 |
| 26 | H | H | F | H | CH₃ | OCH₃ | CF₃ | O | 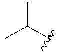 |
| 27 | H | H | F | H | CH₃ | OCH₃ | CF₃ | O | 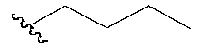 |
| 28 | H | H | F | H | CH₃ | OCH₃ | CF₃ | O | 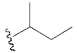 |
| 29 | H | H | F | H | CH₃ | OCH₃ | CF₃ | O | 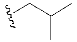 |
| 30 | H | H | F | H | CH₃ | OCH₃ | CF₃ | O | 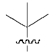 |
| 31 | H | H | F | H | CH₃ | OCH₃ | CF₃ | O | 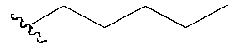 |
| 32 | H | H | F | H | CH₃ | OCH₃ | CF₃ | O | 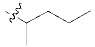 |
| 33 | H | H | F | H | CH₃ | OCH₃ | CF₃ | O | 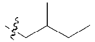 |
| 34 | H | H | F | H | CH₃ | OCH₃ | CF₃ | O | 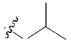 |
| 35 | H | H | F | H | CH₃ | OCH₃ | CF₃ | O | 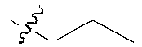 |
| 36 | H | H | F | H | CH₃ | OCH₃ | CF₃ | O | 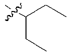 |
| 37 | H | H | F | H | CH₃ | OCH₃ | CF₃ | O | 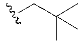 |
| 38 | H | H | F | H | CH₃ | OCH₃ | CF₃ | O | 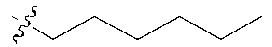 |
| 39 | H | H | F | H | CH₃ | OCH₃ | CF₃ | O | 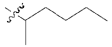 |
| 40 | H | H | F | H | CH₃ | OCH₃ | CF₃ | O | 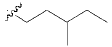 |
| 41 | H | H | F | H | CH₃ | OCH₃ | CF₃ | O | 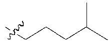 |
| 42 | H | H | F | H | CH₃ | OCH₃ | CF₃ | O | 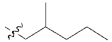 |
| 43 | H | H | F | H | CH₃ | OCH₃ | CF₃ | O | 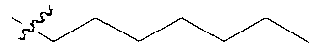 |
| 44 | H | H | F | H | CH₃ | OCH₃ | CF₃ | O | 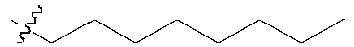 |
| 45 | H | H | F | H | CH₃ | OCH₃ | CF₃ | O | 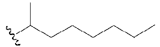 |
| 46 | H | H | F | H | CH₃ | OCH₃ | CF₃ | O | 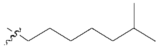 |
| 47 | H | H | F | H | CH₃ | OCH₃ | CF₃ | O | 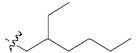 |
| 48 | H | H | F | H | CH₃ | OCH₃ | CF₃ | O | 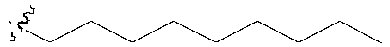 |
| 49 | H | H | F | H | CH₃ | OCH₃ | CF₃ | O | 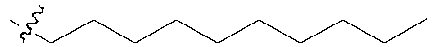 |
| 50 | H | H | F | H | CH₃ | OCH₃ | CF₃ | O | 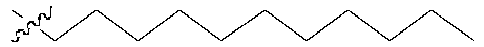 |
| 51 | H | H | F | H | CH₃ | OCH₃ | CF₃ | O | 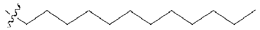 |
| 52 | H | H | F | H | CH₃ | OCH₃ | CF₃ | O | 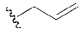 |
| 53 | H | H | F | H | CH₃ | OCH₃ | CF₃ | O | 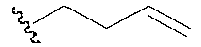 |
| 54 | H | H | F | H | CH₃ | OCH₃ | CF₃ | O | 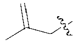 |
| 55 | H | H | F | H | CH₃ | OCH₃ | CF₃ | O | 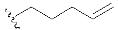 |
| 56 | H | H | F | H | CH₃ | OCH₃ | CF₃ | O | 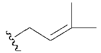 |
| 57 | H | H | F | H | CH₃ | OCH₃ | CF₃ | O | 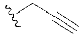 |
| 58 | H | H | F | H | CH₃ | OCH₃ | CF₃ | O | 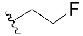 |
| 59 | H | H | F | H | CH₃ | OCH₃ | CF₃ | O | 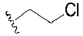 |
| 60 | H | H | F | H | CH₃ | OCH₃ | CF₃ | O | 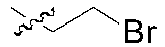 |
| 61 | H | H | F | H | CH₃ | OCH₃ | CF₃ | O | 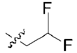 |
| 62 | H | H | F | H | CH₃ | OCH₃ | CF₃ | O | 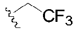 |
| 63 | H | H | F | H | CH₃ | OCH₃ | CF₃ | O | 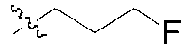 |
| 64 | H | H | F | H | CH₃ | OCH₃ | CF₃ | O | 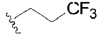 |
| 65 | H | H | F | H | CH₃ | OCH₃ | CF₃ | O | 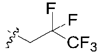 |
| 66 | H | H | F | H | CH₃ | OCH₃ | CF₃ | O | 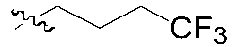 |
| 67 | H | H | F | H | CH₃ | OCH₃ | CF₃ | O | 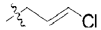 |
| 68 | H | H | F | H | CH₃ | OCH₃ | CF₃ | O | 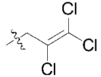 |
| 69 | H | H | F | H | CH₃ | OCH₃ | CF₃ | O | 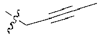 |
| 70 | H | H | F | H | CH₃ | OCH₃ | CF₃ | O | 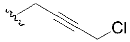 |
| 71 | H | H | F | H | CH₃ | OCH₃ | CF₃ | O | 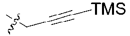 |
| 72 | H | H | F | H | CH₃ | OCH₃ | CF₃ | O | 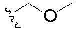 |
| 73 | H | H | F | H | CH₃ | OCH₃ | CF₃ | O | 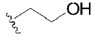 |
| 74 | H | H | F | H | CH₃ | OCH₃ | CF₃ | O | 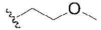 |
| 75 | H | H | F | H | CH₃ | OCH₃ | CF₃ | O | 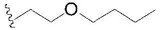 |
| 76 | H | H | F | H | CH₃ | OCH₃ | CF₃ | O | 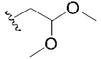 |
| 77 | H | H | F | H | CH₃ | OCH₃ | CF₃ | O | 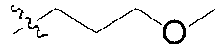 |
| 78 | H | H | F | H | CH₃ | OCH₃ | CF₃ | O | 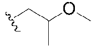 |
| 79 | H | H | F | H | CH₃ | OCH₃ | CF₃ | O | 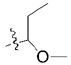 |
| 80 | H | H | F | H | CH₃ | OCH₃ | CF₃ | O | 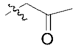 |
| 81 | H | H | F | H | CH₃ | OCH₃ | CF₃ | O | 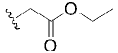 |
| 82 | H | H | F | H | CH₃ | OCH₃ | CF₃ | O | 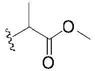 |
| 83 | H | H | F | H | CH₃ | OCH₃ | CF₃ | O | 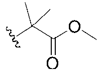 |
| 84 | H | H | F | H | CH₃ | OCH₃ | CF₃ | O | 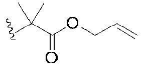 |
| 85 | H | H | F | H | CH₃ | OCH₃ | CF₃ | O | 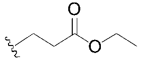 |
| 86 | H | H | F | H | CH₃ | OCH₃ | CF₃ | O | 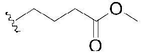 |
| 87 | H | H | F | H | CH₃ | OCH₃ | CF₃ | O | 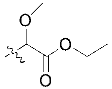 |
| 88 | H | H | F | H | CH₃ | OCH₃ | CF₃ | O | 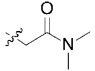 |
| 89 | H | H | F | H | CH₃ | OCH₃ | CF₃ | O | 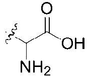 |
| 90 | H | H | F | H | CH₃ | OCH₃ | CF₃ | O | 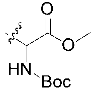 |
| 91 | H | H | F | H | CH₃ | OCH₃ | CF₃ | O | 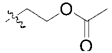 |
| 92 | H | H | F | H | CH₃ | OCH₃ | CF₃ | O | 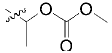 |
| 93 | H | H | F | H | CH₃ | OCH₃ | CF₃ | O | 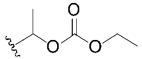 |
| 94 | H | H | F | H | CH₃ | OCH₃ | CF₃ | O | 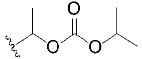 |
| 95 | H | H | F | H | CH₃ | OCH₃ | CF₃ | O | 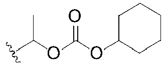 |
| 96 | H | H | F | H | CH₃ | OCH₃ | CF₃ | O | 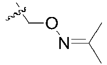 |
| 97 | H | H | F | H | CH₃ | OCH₃ | CF₃ | O | 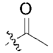 |
| 98 | H | H | F | H | CH₃ | OCH₃ | CF₃ | O | 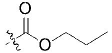 |
| 99 | H | H | F | H | CH₃ | OCH₃ | CF₃ | O | 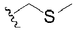 |
| 100 | H | H | F | H | CH₃ | OCH₃ | CF₃ | O | 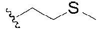 |
| 101 | H | H | F | H | CH₃ | OCH₃ | CF₃ | O | 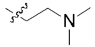 |
| 102 | H | H | F | H | CH₃ | OCH₃ | CF₃ | O | 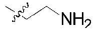 |
| 103 | H | H | F | H | CH₃ | OCH₃ | CF₃ | O | 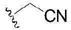 |
| 104 | H | H | F | H | CH₃ | OCH₃ | CF₃ | O | 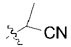 |
| 105 | H | H | F | H | CH₃ | OCH₃ | CF₃ | O | 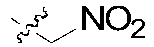 |
| 106 | H | H | F | H | CH₃ | OCH₃ | CF₃ | O | 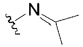 |
| 107 | H | H | F | H | CH₃ | OCH₃ | CF₃ | O | 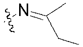 |
| 108 | H | H | F | H | CH₃ | OCH₃ | CF₃ | O | 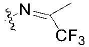 |
| 109 | H | H | F | H | CH₃ | OCH₃ | CF₃ | O | 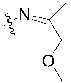 |
| 110 | H | H | F | H | CH₃ | OCH₃ | CF₃ | O | 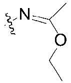 |
| 111 | H | H | F | H | CH₃ | OCH₃ | CF₃ | O | 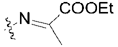 |
| 112 | H | H | F | H | CH₃ | OCH₃ | CF₃ | O | 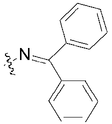 |
| 113 | H | H | F | H | CH₃ | OCH₃ | CF₃ | O | 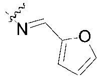 |
| 114 | H | H | F | H | CH₃ | OCH₃ | CF₃ | O | 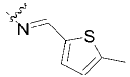 |
| 115 | H | H | F | H | CH₃ | OCH₃ | CF₃ | O | 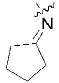 |
| 116 | H | H | F | H | CH₃ | OCH₃ | CF₃ | O | 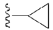 |
| 117 | H | H | F | H | CH₃ | OCH₃ | CF₃ | O | 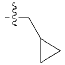 |
| 118 | H | H | F | H | CH₃ | OCH₃ | CF₃ | O | 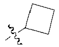 |
| 119 | H | H | F | H | CH₃ | OCH₃ | CF₃ | O | 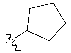 |
| 120 | H | H | F | H | CH₃ | OCH₃ | CF₃ | O | 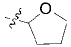 |
| 121 | H | H | F | H | CH₃ | OCH₃ | CF₃ | O | 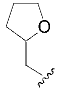 |
| 122 | H | H | F | H | CH₃ | OCH₃ | CF₃ | O | 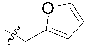 |
| 123 | H | H | F | H | CH₃ | OCH₃ | CF₃ | O | 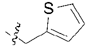 |
| 124 | H | H | F | H | CH₃ | OCH₃ | CF₃ | O | 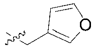 |
| 125 | H | H | F | H | CH₃ | OCH₃ | CF₃ | O | 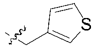 |
| 126 | H | H | F | H | CH₃ | OCH₃ | CF₃ | O | 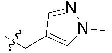 |
| 127 | H | H | F | H | CH₃ | OCH₃ | CF₃ | O | 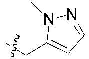 |
| 128 | H | H | F | H | CH₃ | OCH₃ | CF₃ | O | 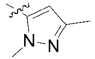 |
| 129 | H | H | F | H | CH₃ | OCH₃ | CF₃ | O | 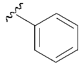 |
| 130 | H | H | F | H | CH₃ | OCH₃ | CF₃ | O | 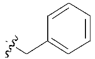 |
| 131 | H | H | F | H | CH₃ | OCH₃ | CF₃ | O | 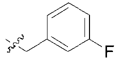 |
| 132 | H | H | F | H | CH₃ | OCH₃ | CF₃ | O | 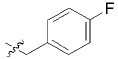 |
| 133 | H | H | F | H | CH₃ | OCH₃ | CF₃ | O | 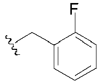 |
| 134 | H | H | F | H | CH₃ | OCH₃ | CF₃ | O | 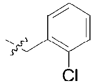 |
| 135 | H | H | F | H | CH₃ | OCH₃ | CF₃ | O | 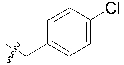 |
| 136 | H | H | F | H | CH₃ | OCH₃ | CF₃ | O | 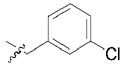 |
| 137 | H | H | F | H | CH₃ | OCH₃ | CF₃ | O | 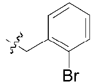 |
| 138 | H | H | F | H | CH₃ | OCH₃ | CF₃ | O | 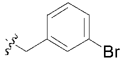 |
| 139 | H | H | F | H | CH₃ | OCH₃ | CF₃ | O | 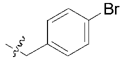 |
| 140 | H | H | F | H | CH₃ | OCH₃ | CF₃ | O | 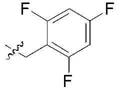 |
| 141 | H | H | F | H | CH₃ | OCH₃ | CF₃ | O | 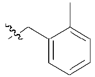 |
| 142 | H | H | F | H | CH₃ | OCH₃ | CF₃ | O | 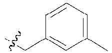 |
| 143 | H | H | F | H | CH₃ | OCH₃ | CF₃ | O | 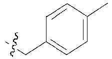 |
| 144 | H | H | F | H | CH₃ | OCH₃ | CF₃ | O | 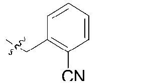 |
| 145 | H | H | F | H | CH₃ | OCH₃ | CF₃ | O | 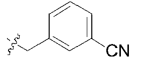 |
| 146 | H | H | F | H | CH₃ | OCH₃ | CF₃ | O | 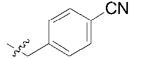 |
| 147 | H | H | F | H | CH₃ | OCH₃ | CF₃ | O | 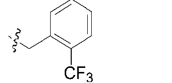 |
| 148 | H | H | F | H | CH₃ | OCH₃ | CF₃ | O | 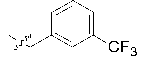 |
| 149 | H | H | F | H | CH₃ | OCH₃ | CF₃ | O | 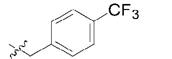 |
| 150 | H | H | F | H | CH₃ | OCH₃ | CF₃ | O | 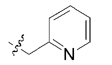 |
| 151 | H | H | F | H | CH₃ | OCH₃ | CF₃ | O | 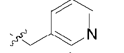 |
| 152 | H | H | F | H | CH₃ | OCH₃ | CF₃ | O | 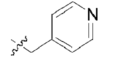 |
| 153 | H | H | F | H | CH₃ | OCH₃ | CF₃ | O | 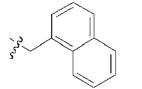 |
| 154 | H | H | F | H | CH₃ | OCH₃ | CF₃ | O | 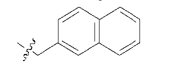 |
| 155 | H | H | F | H | CH₃ | OCH₃ | CF₃ | S | CH₃ |
| 156 | H | H | F | H | CH₃ | OCH₃ | CF₃ | S | CH₂CH₃ |
| 157 | H | H | F | H | CH₃ | OCH₃ | CF₃ | S | 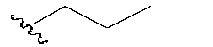 |
| 158 | H | H | F | H | CH₃ | OCH₃ | CF₃ | S | 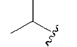 |
| 159 | H | H | F | H | CH₃ | OCH₃ | CF₃ | S | 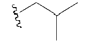 |
| 160 | H | H | F | H | CH₃ | OCH₃ | CF₃ | S | 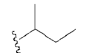 |
| 161 | H | H | F | H | CH₃ | OCH₃ | CF₃ | S | 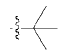 |
| 162 | H | H | F | H | CH₃ | OCH₃ | CF₃ | S | 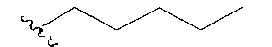 |
| 163 | H | H | F | H | CH₃ | OCH₃ | CF₃ | S | 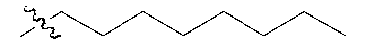 |
| 164 | H | H | F | H | CH₃ | OCH₃ | CF₃ | S | 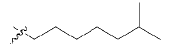 |
| 165 | H | H | F | H | CH₃ | OCH₃ | CF₃ | S | 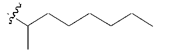 |
| 166 | H | H | F | H | CH₃ | OCH₃ | CF₃ | S | 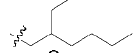 |
| 167 | H | H | F | H | CH₃ | OCH₃ | CF₃ | S | 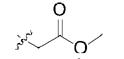 |
| 168 | H | H | F | H | CH₃ | OCH₃ | CF₃ | S | 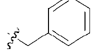 |
| 169 | H | H | F | H | CH₃ | OCH₃ | CF₃ | S | 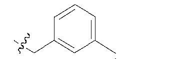 |
| 170 | H | H | F | H | CH₃ | OCH₃ | CF₃ | S | 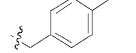 |
| 171 | H | H | F | H | CH₃ | OCH₃ | CF₃ | S | 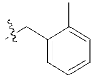 |
| 172 | H | H | F | H | CH₃ | OCH₃ | CF₃ | S | 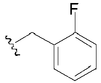 |
| 173 | H | H | F | H | CH₃ | OCH₃ | CF₃ | S | 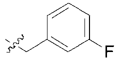 |
| 174 | H | H | F | H | CH₃ | OCH₃ | CF₃ | S | 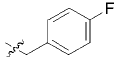 |
| 175 | H | H | F | H | CH₃ | OCH₃ | CF₃ | S | 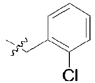 |
| 176 | H | H | F | H | CH₃ | OCH₃ | CF₃ | S | 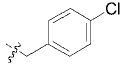 |
| 177 | H | H | F | H | CH₃ | OCH₃ | CF₃ | S | 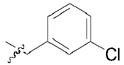 |
| 178 | H | H | F | H | CH₃ | OCH₃ | CF₃ | S | 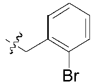 |
| 179 | H | H | F | H | CH₃ | OCH₃ | CF₃ | S | 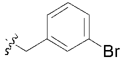 |
| 180 | H | H | F | H | CH₃ | OCH₃ | CF₃ | S | 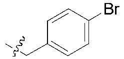 |
| 181 | H | H | F | H | CH₃ | OCH₃ | CF₃ | S | 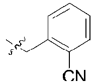 |
| 182 | H | H | F | H | CH₃ | OCH₃ | CF₃ | S | 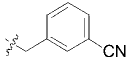 |
| 183 | H | H | F | H | CH₃ | OCH₃ | CF₃ | S | 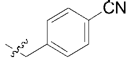 |
| 184 | H | H | F | H | CH₃ | OCH₃ | CF₃ | S | 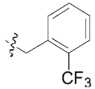 |
| 185 | H | H | F | H | CH₃ | OCH₃ | CF₃ | S | 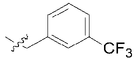 |
| 186 | H | H | F | H | CH₃ | OCH₃ | CF₃ | S | 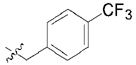 |
| 187 | H | H | F | H | CH₃ | OCH₃ | CF₃ | S | 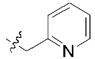 |
| 188 | H | H | F | H | CH₃ | OCH₃ | CF₃ | S | 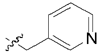 |
| 189 | H | H | F | H | CH₃ | OCH₃ | CF₃ | S | 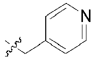 |
| 190 | H | H | F | H | CH₃ | OCH₃ | CF₃ | S | 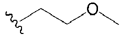 |
| 191 | H | H | F | H | CH₃ | OCH₃ | CF₃ | O | 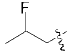 |
| 192 | H | H | F | H | CH₃ | OCH₃ | CF₃ | O | 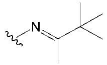 |
| 193 | H | H | F | H | CH₃ | OCH₃ | CF₃ | O | 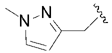 |
| 194 | H | H | F | H | CH₃ | OCH₃ | 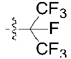 | O | CH₃ |
| 195 | H | H | F | H | CH₃ | OCH₃ | 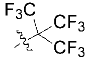 | O | CH₂CH₃ |
| 196 | H | H | F | H | CH₃ | OCH₃ | 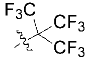 | O | 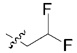 |
| 197 | H | H | F | H | CH₃ | OCH₃ | 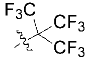 | O | 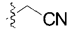 |
| 198 | H | H | F | H | CH₃ | OCH₃ | 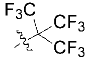 | O | 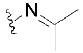 |
| 199 | H | H | F | H | CH₃ | OCH₃ | CF₃ | O | K |
| 200 | H | H | F | H | CH₃ | OCH₃ | CF₃ | O | Na |
| 201 | H | H | F | H | CH₃ | OCH₃ | Br | O | CH₃ |
| 202 | H | H | F | H | CH₃ | OCH₃ | Br | O | CH₂CH₃ |
| 203 | H | H | F | H | CH₃ | OCH₃ | Br | O | 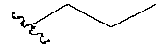 |
| 204 | H | H | F | H | CH₃ | OCH₃ | Br | O | 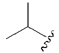 |
| 205 | H | H | F | H | CH₃ | OCH₃ | Br | O | 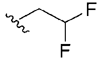 |
| 206 | H | H | F | H | CH₃ | OCH₃ | Br | O | 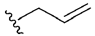 |
| 207 | H | H | F | H | CH₃ | OCH₃ | Br | O | 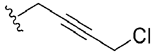 |
| 208 | H | H | F | H | CH₃ | OCH₃ | Br | O | 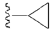 |
| 209 | H | H | F | H | CH₃ | OCH₃ | Br | O | 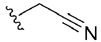 |
| 210 | H | H | F | H | CH₃ | OCH₃ | Br | O | 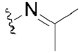 |
| 211 | H | H | F | H | CH₃ | OCH₃ | Br | O | 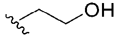 |
| 212 | H | H | F | H | CH₃ | OCH₃ | Br | O | 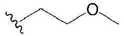 |
| 213 | H | H | F | H | CH₃ | OCH₃ | Br | O | 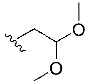 |
| 214 | H | H | F | H | CH₃ | OCH₃ | Br | O | 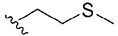 |
| 215 | H | H | F | H | CH₃ | OCH₃ | Br | O | 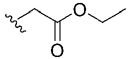 |
| 216 | H | H | F | H | CH₃ | OCH₃ | Br | O | 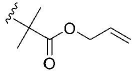 |
| 217 | H | H | F | H | CH₃ | OCH₃ | Br | O | 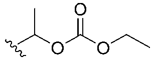 |
| 218 | H | H | F | H | CH₃ | OCH₃ | Br | O | 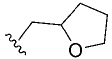 |
| 219 | H | H | F | H | CH₃ | OCH₃ | Br | O | 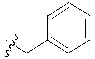 |
| 220 | H | H | F | H | CH₃ | OCH₃ | Br | O | 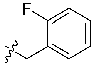 |
| 221 | H | H | F | H | CH₃ | OCH₃ | Br | S | CH₃ |
| 222 | H | H | F | H | CH₃ | OCH₃ | Br | S | CH₂CH₃ |
| 223 | H | H | F | H | 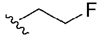 | OCH₃ | Br | O | CH₃ |
| 224 | H | H | F | H | CH₃ | OCH₃ | 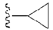 | O | CH₃ |
| 225 | H | H | F | H | CH₃ | OCH₃ | 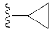 | O | CH₂CH₃ |
| 226 | H | H | F | H | CH₃ | OCH₃ | 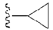 | O | 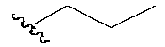 |
| 227 | H | H | F | H | CH₃ | OCH₃ | 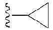 | O | 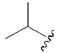 |
| 228 | H | H | F | H | CH₃ | OCH₃ | 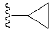 | O | 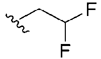 |
| 229 | H | H | F | H | CH₃ | OCH₃ | 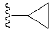 | O | 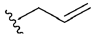 |
| 230 | H | H | F | H | CH₃ | OCH₃ | 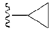 | O | 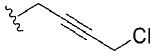 |
| 231 | H | H | F | H | CH₃ | OCH₃ | 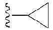 | O | 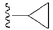 |
| 232 | H | H | F | H | CH₃ | OCH₃ | 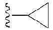 | O | 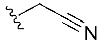 |
| 233 | H | H | F | H | CH₃ | OCH₃ | 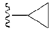 | O | 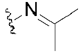 |
| 234 | H | H | F | H | CH₃ | OCH₃ | 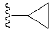 | O | 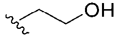 |
| 235 | H | H | F | H | CH₃ | OCH₃ | 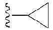 | O | 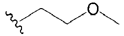 |
| 236 | H | H | F | H | CH₃ | OCH₃ | 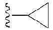 | O | 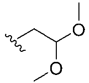 |
| 237 | H | H | F | H | CH₃ | OCH₃ | 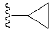 | O | 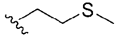 |
| 238 | H | H | F | H | CH₃ | OCH₃ | 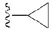 | O | 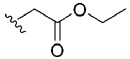 |
| 239 | H | H | F | H | CH₃ | OCH₃ | 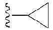 | O | 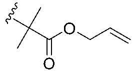 |
| 240 | H | H | F | H | CH₃ | OCH₃ | 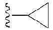 | O | 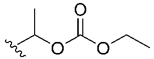 |
| 241 | H | H | F | H | CH₃ | OCH₃ | 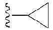 | O | 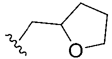 |
| 242 | H | H | F | H | CH₃ | OCH₃ | 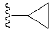 | O | 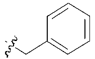 |
| 243 | H | H | F | H | CH₃ | OCH₃ | 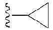 | O | 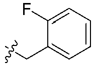 |
| 244 | H | H | F | H | CH₃ | OCH₃ | 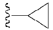 | S | CH₃ |
| 245 | H | H | F | H | CH₃ | OCH₃ | 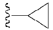 | S | CH₂CH₃ |
| 246 | H | H | F | H | 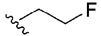 | OCH₃ | 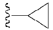 | O | CH₃ |
| 247 | H | H | F | H | CH₃ | OCHF₂ | CF₃ | O | CH₃ |

**Table 2 ¹H NMR of Compounds**

| **No.** | **¹H NMR** |
|---|---|
| 1 | ¹H NMR (300 MHz, Chloroform-*d*) δ 7.81 - 7.76 (m, 1H), 7.41 (d, *J* = 8.4 Hz, 1H), 5.83 (s, 2H), 4.22 - 3.90 (m, 6H). |
| 2 | ¹H NMR (300 MHz, Chloroform-*d*) δ 7.53 (d, *J =* 8.1 Hz, 1H), 7.41 (d, *J =* 8.1 Hz, 1H), 5.66 (s, 2H), 3.96 (s, 3H), 3.95 (s, 3H), 3.94 (s, 3H). |
| 3 | ¹H NMR (300 MHz, DMSO-*d*₆) δ 7.64 - 7.51 (m, 4H), 3.95 (s, 3H), 3.87 (s, 3H), 3.74 (s, 3H). |
| 4 | ¹H NMR (300 MHz, Chloroform-*d*) δ 7.74 - 7.66 (m, 1H), 7.42 (d, *J =* 8.1 Hz, 1H), 6.96 (t, *J* = 54.0 Hz, 1H), 5.51 (s, 2H), 4.08-4.06 - 3.96 (m, 9H). |
| 21 | ¹H NMR (300 MHz, Chloroform-*d*) δ 7.67 - 7.65 (m, 1H), 7.37 (d, *J* = 8.4 Hz, 1H), 5.57 (s, 2H), 4.55 (t, *J =* 5.0 Hz, 2H), 4.02 (s, 3H), 3.95 (s, 3H), 3.78 (t, *J =* 5.0 Hz, 2H), 3.57 (q, *J* = 7.2 Hz, 2H), 1.21 (t, *J* = 7.2 Hz, 3H). |
| 22 | ¹H NMR (300 MHz, Chloroform-*d*) δ 7.68 - 7.66 (m, 1H), 7.37 (d, *J* = 8.4 Hz, 1H), 5.48 (s, 2H), 4.62 - 4.48 (m, 2H), 4.08 - 3.98 (m, 3H), 3.99 - 3.90 (m, 3H), 3.84 - 3.72 (m, 2H), 3.52 - 3.40 (m, 2H), 1.66 - 1.52 (m, 2H), 0.99 - 0.85 (m, 3H). |
| 23 | ¹H NMR (300 MHz, Chloroform-*d*) δ 7.70 - 7.60 (m, 1H), 7.39 - 7.36 (d, *J* = 7.8 Hz, 1H), 5.47 (s, 2H), 3.95 (s, 3H), 3.75 (s, 3H), 4.59 - 4.56 (m, 2H), 3.87 - 3.84 (t, *J* = 4.8 Hz, 2H), 3.71 - 3.68 (t, *J* = 4.8 Hz, 2H), 3.56 - 3.53 (t, *J* = 4.5 Hz, 2H), 3.37 (s, 3H). |
| 24 | ¹H NMR (300 MHz, Chloroform-*d*) δ 7.71 - 7.65 (m, 1H), 7.38 (d, *J =* 8.7 Hz, 1H), 5.43 (s, 2H), 4.48 (q, *J =* 7.5 Hz, 2H), 4.05 - 4.01 (m, 3H) , 3.95 - 3.94 (m, 3H), 1.44 (t, *J* = 7.5 Hz, 3H). |
| 25 | ¹H NMR (300 MHz, Chloroform-d) δ 7.72 - 7.62 (m, 1H), 7.37 (d, *J* = 8.4 Hz, 1H), 5.44 (s, 2H), 4.37 (t, *J* = 6.9 Hz, 2H), 4.03 (d, *J =* 1.8 Hz, 3H), 3.94 (d, *J =* 1.8 Hz, 3H), 1.89 - 1.74 (m, 2H), 1.03 (t, *J =* 7.5 Hz, 3H). |
| 26 | ¹H NMR (300 MHz, Chloroform-*d*) δ 7.73 - 7.63 (m, 1H), 7.37 (d, *J* = 8.4 Hz, 1H), 5.53 (s, 2H), 5.41 - 5.26 (m, 1H), 4.08 - 3.99 (m, 3H), 3.96 - 3.87 (m, 3H), 1.42 (d, *J* = 6.3 Hz, 6H). |
| 52 | ¹H **NMR** (300 MHz, Chloroform-*d*) δ 7.67 - 7.65 (m, 1H), 7.37 (d, *J* = 8.4 Hz, 1H), 6.02 - 6.01 (m, 1H), 5.48 - 5.33 (m, 4H), 4.90 - 4.88 (m, 2H), 4.03 - 4.01 (m, 3H), 3.98 (s, 3H). |
| 57 | ¹H NMR(300 MHz, Chloroform-d) δ 7.69 - 7.64 (m, 1H), 7.38 (d, *J =* 8.4 Hz, 1H), 5.47 (s, 2H), 4.98 - 4.95 (m, 2H), 4.03 - 4.01 (m, 3H), 3.97 (s, 3H), 2.55 - 2.54 (m, 1H) |
| 58 | ¹H NMR (300 MHz, Chloroform-*d*) δ 7.70 - 7.67 (m, 1H), 7.38 (d, *J* = 8.4 Hz, 1H), 5.47 (s, 2H), 4.86 - 4.67 (m, 4H), 4.04 (s, 3H), 3.96 (s, 3H). |
| 59 | ¹H NMR (300 MHz, Chloroform-*d*) δ 7.73 - 7.63 (m, 1H), 7.40 (d, *J* = 8.4 Hz, 1H), 5.47 (s, 2H), 4.67 (t, *J =* 5.7 Hz, 2H), 4.07 - 3.94 (m, 6H), 3.85 (t, *J* = 5.7 Hz, 2H). |
| 60 | ¹H NMR (300 MHz, Chloroform-*d*) δ 7.69 - 7.58 (m, 1H), 7.36 (d, *J* = 8.4 Hz, 1H), 5.76 (s, 2H), 4.70 (t, *J* = 6.9 Hz, 2H), 4.00 (d, *J =* 1.8 Hz, 3H), 3.97 - 3.87 (m, 3H), 3.65 (dd, *J* = 6.9 Hz, 2H). |
| 61 | ¹H NMR (300 MHz, Chloroform-*d*) δ 7.76 - 7.66 (m, 1H), 7.38 (d, *J* = 8.1 Hz, 1H), 6.34 - 5.90 (m, 1H), 5.59 (s, 2H), 4.60 (d, *J =* 3.9 Hz, 2H), 4.03 (s, 3H), 3.93 (s, 3H). |
| 62 | ¹H NMR (300 MHz, Chloroform-*d*) δ 7.74 - 7.66 (m, 1H), 7.38 (d, *J* = 8.7 Hz, 1H), 5.50 (s, 2H), 4.79 - 4.75 (m, 2H), 4.05 - 4.01 (m, 3H) , 3.95 - 3.94 (m, 3H). |
| 63 | ¹H NMR (300 MHz, Chloroform-*d*) δ 7.72 - 7.67 (m, 1H), 7.38 (d, *J* = 8.4 Hz, 1H), 5.52 (s, 2H), 4.77 - 4.47 (m, 4H), 4.03 (d, *J =* 1.8 Hz, 3H), 3.94 (s, 3H), 2.33 - 2.10 (m, 2H). |
| 64 | ¹H NMR (300 MHz, Chloroform-*d*) δ 7.76 - 7.66 (m, 1H), 7.37 (d, *J* = 8.4 Hz, 1H), 5.47 (s, 2H), 4.66 - 4.55 (m, 2H), 4.02 (t, *J =* 1.2 Hz, 3H), 3.93 (d, *J =* 1.2 Hz, 3H), 2.74 - 2.56 (m, 2H). |
| 72 | ¹H NMR (300 MHz, Chloroform-d) δ 7.76 - 7.68 (m, 1H), 7.38 (d, *J* = 8.4 Hz, 1H), 5.58 - 5.43 (m, 4H), 4.07 - 3.94 (m, 6H), 3.63 - 3.56 (m, 3H). |
| 73 | ¹H NMR (300 MHz, Chloroform-*d*) δ 7.62 - 7.52 (m, 1H), 7.35 (d, *J* = 8.4 Hz, 1H), 5.74 (s, 2H), 4.49 - 4.38 (m, 2H), 4.03 - 3.96 (m, 3H), 3.92 - 3.88 (m, 3H), 3.87 - 3.80 (m, 2H), 2.85 - 2.57 (m, 1H). |
| 74 | ¹H NMR (300 MHz, Chloroform-*d*) δ 7.76 - 7.60 (m, 1H), 7.37 (d, *J* = 8.1 Hz, 1H), 5.48 (s, 2H), 4.64 - 4.47 (m, 2H), 4.07 - 3.89 (m, 6H), 3.82 - 3.69 (m, 2H), 3.42 (s, 3H). |
| 76 | ¹H NMR (300 MHz, Chloroform-*d*) δ 7.70 - 7.61 (m, 1H), 7.36 (d, *J* = 8.4 Hz, 1H), 5.57 (s, 2H), 4.77 - 4.69 (m, 1H), 4.42 (dd, *J* = 5.4, 0.8 Hz, 2H), 4.04 - 3.99 (m, 3H), 3.93 (d, *J* = 0.8 Hz, 3H), 3.44 - 3.40 (m, 6H). |
| 81 | ¹H NMR (300 MHz, Chloroform-*d*) δ 7.76 - 7.59 (m, 1H), 7.39 (d, *J =* 8.4 Hz, 1H), 5.51 (s, 2H), 4.92 (s, 2H), 4.27 (q, *J =* 7.2 Hz, 2H), 4.07 - 3.98 (m, 6H), 1.29 (t, *J =* 7.2 Hz, 3H). |
| 82 | ¹H NMR (300 MHz, Chloroform-*d*) δ 7.76 - 7.65 (m, 1H), 7.40 (d, *J* = 8.4 Hz, 1H), 5.52 - 5.27 (m, 3H), 4.07 - 3.96 (m, 9H), 3.80 (d, *J* = 3.6 Hz, 3H). |
| 93 | ¹H NMR (300 MHz, Chloroform-*d*) 7.71 - 7.65 (m, 1H), 7.38 (d, *J* = 8.4 Hz, 1H), 7.05 (q, *J* = 5.7 Hz, 1H), 5.46 (s, 2H), 4.25 (q, *J =* 7.2 Hz, 2H), 4.07 - 4.02 (m, 3H), 3.96 (s, 3H), 1.69 (d, *J =* 5.7 Hz, 3H), 1.31 (d, *J =* 7.2 Hz, 3H). |
| 100 | ¹H NMR (300 MHz, Chloroform-*d*) δ 7.74 - 7.60 (m, 1H), 7.38 (d, *J* = 8.4 Hz, 1H), 5.44 (s, 2H), 4.58 (t, *J* = 6.6 Hz, 2H), 4.08 - 4.00 (m, 3H), 3.97 (s, 3H), 2.88 (q, *J* = 6.6 Hz, 2H), 2.27 - 2.17 (m, 3H). |
| 103 | ¹H NMR (300 MHz, Chloroform-*d*) δ 7.69 - 7.57 (m, 1H), 7.39 (d, *J* = 8.4 Hz, 1H), 5.68 (s, 2H), 5.07 - 4.84 (m, 2H), 4.03 (d, *J =* 1.2 Hz, 3H), 3.98 - 3.93 (m, 3H). |
| 106 | ¹H NMR (300 MHz, Chloroform-*d*) δ 7.74 - 7.66 (m, 1H), 7.38 (d, *J* = 8.7 Hz, 1H), 5.50 (s, 2H), 4.08 - 4.01 (m, 3H), 4.01 - 3.90 (m, 3H), 2.13 (s, 6H). |
| 116 | ¹H NMR (300 MHz, Chloroform-d) 7.71 - 7.65 (m, 1H), 7.38 (d, *J* = 8.7 Hz, 1H), 5.42 (s, 2H), 4.50 - 4.41 (m, 1H), 4.05 - 4.01 (m, 3H), 3.98 - 3.92 (m, 3H), 0.92 - 0.85 (m, 4H). |
| 117 | ¹H NMR (300 MHz, Chloroform-*d*) δ 7.72 - 7.63 (m, 1H), 7.37 (d, *J* = 8.4 Hz, 1H), 5.49 (s, 2H), 4.25 (d, *J =* 7.5 Hz, 2H), 4.02 (d, *J =* 1.8 Hz, 3H), 3.96 (q, *J =* 0.6 Hz, 3H), 1.36 - 1.24 (m, 1H), 0.70 - 0.57 (m, 2H), 0.46 - 0.36 (m, 2H). |
| 118 | ¹H NMR (300 MHz, Chloroform-*d*) δ 7.70 - 7.67 (m, 1H), 7.38 (d, *J* = 8.4 Hz, 1H), 5.44 (s, 2H), 5.31 - 5.26 (m, 1H), 4.03 (d, *J* = 1.8 Hz, 3H), 3.95 (s, 3H), 2.52 - 2.45 (m, 2H), 2.34 - 2.21 (m, 2H), 1.94 - 1.83 (m, 1H), 1.76 - 1.67 (m, 1H). |
| 119 | ¹H NMR (300 MHz, Chloroform-*d*) δ 7.73 - 7.64 (m, 1H), 7.38 (d, *J* = 8.4 Hz, 1H), 5.52 - 5.42 (m, 3H), 4.05 - 4.01 (m, 3H), 3.94 - 3.90 (m, 3H), 2.05 - 1.89 (m, 4H), 1.84 - 1.73 (m, 2H), 1.70 - 1.58 (m, 2H). |
| 121 | ¹H NMR (300 MHz, Chloroform-*d*) δ 7.70 - 7.67 (m, 1H), 7.38 (d, *J* = 8.4 Hz, 1H), 5.45 (s, 2H), 4.48 - 4.27 (m, 3H), 4.03 (s, 3H), 4.01 - 3.80 (m, 5H), 2.05 - 1.94 (m, 3H), 1.80 - 1.74 (m, 1H). |
| 129 | ¹H NMR (300 MHz, Chloroform-*d*) δ 7.74 (t, *J =* 7.5 Hz, 1H), 7.52 - 7.38 (m, 3H), 7.35 - 7.28 (m, 3H), 5.50 (s, 2H), 4.12 - 3.94 (m, 6H). |
| 130 | ¹H NMR (300 MHz, Chloroform-*d*) δ 7.72 - 7.62 (m, 1H), 7.40 (d, *J* = 8.4 Hz, 1H), 7.41 - 7.32 (m, 5H), 5.45 - 5.42 (m, 4H), 4.03 (d, *J* = 1.8 Hz, 3H), 3.81 (s, 3H). |
| 133 | ¹H NMR (300 MHz, Chloroform-*d*) δ 7.68 (t, *J* = 7.8 Hz, 1H), 7.55 (t, *J* = 6.6 Hz, 1H), 7.39 - 7.36 (m, 2H), 7.19 - 7.07 (m, 2H), 5.52 (s, 2H), 5.44 (s, 2H), 4.03 - 4.01 (m, 3H), 3.86 (s, 3H). |
| 150 | ¹H NMR (300 MHz, Chloroform-*d*) δ 8.61 (d, *J* = 4.8 Hz, 1H), 7.76 - 7.68 (m, 2H), 7.56 (d, *J* = 7.5 Hz, 1H), 7.39 (d, *J =* 8.1 Hz, 1H), 7.29 - 7.27 (m, 1H), 5.56 (s, 2H), 5.45 (s, 2H), 4.04 (d, *J =* 1.8 Hz, 3H), 3.94 (s, 3H). |
| 151 | ¹H NMR (300 MHz, Chloroform-*d*) δ 8.74 (s, 1H), 8.66 - 8.54 (m, 1H), 7.83 (d, *J =* 8.1 Hz, 1H), 7.66 (t, *J* = 7.5 Hz, 1H), 7.42 - 7.28 (m, 2H), 5.57 - 5.40 (m, 4H), 4.02 (d, *J* = 1.8 Hz, 3H), 3.87 - 3.49 (m, 3H). |
| 152 | ¹H NMR (300 MHz, Chloroform-*d*) δ 8.65 - 8.63 (m, 2H), 7.73 - 7.70 (m, 1H), 7.46 - 7.35 (m, 3H), 5.60 - 5.33 (m, 4H), 4.05 (d, *J* = 1.8 Hz, 3H), 3.90 (s, 3H). |
| 155 | ¹H NMR (300 MHz, Chloroform-*d*) δ 7.87 - 7.73 (m, 1H), 7.41 (d, *J* = 8.1 Hz, 1H), 5.64 (s, 2H), 4.04 (s, 3H), 3.96 (s, 3H), 2.41 (s, 3H). |
| 156 | ¹H NMR (300 MHz, Chloroform-*d*) δ 7.82 - 7.77 (m, 1H), 7.39 (d, *J* = 8.4 Hz, 1H), 5.53 (s, 2H), 4.05 (d, *J =* 1.5 Hz, 3H), 3.96 (s, 3H), 3.01 (q, *J =* 7.5 Hz, 2H), 1.37 (d, *J =* 7.5 Hz, 3H). |
| 157 | ¹H NMR (300 MHz, Chloroform-*d*) δ 7.82 - 7.79 (m, 1H), 7.41 (d, *J* = 8.4 Hz, 1H), 5.53 (s, 2H), 4.05 - 4.03 (m, 3H), 3.95 (s, 3H), 2.98 (t, *J* = 7.5 Hz, 2H), 1.75 - 1.67 (m, 2H), 1.04 (t, *J* = 7.5 Hz, 3H). |
| 190 | ¹H NMR (300 MHz, Chloroform-*d*) δ 7.86 - 7.74 (m, 1H), 7.40 (d, *J* = 8.4 Hz, 1H), 5.70 (s, 2H), 4.10 - 3.98 (m, 3H), 3.94 (s, 3H), 3.61 (t, *J* = 6.5 Hz, 2H), 3.44 - 3.32 (m, 3H), 3.21 (t, *J* = 6.5 Hz, 2H). |
| 191 | ¹H NMR (300 MHz, Chloroform-*d*) δ 7.76 - 7.60 (m, 1H), 7.37 (d, *J* = 8.4 Hz, 1H), 5.59 (s, 2H), 5.18 - 4.84 (m, 1H), 4.60 - 4.36 (m, 2H), 4.09 - 3.97 (m, 3H), 3.94 (s, 3H), 1.54 - 1.34 (m, 3H). |
| 192 | ¹H NMR (300 MHz, Chloroform-*d*) δ 7.78 - 7.61 (m, 1H), 7.37 (d, *J* = 8.4 Hz, 1H), 5.61 (s, 2H), 4.04 - 3.93 (m, 6H), 2.07 - 2.02 (m, 3H), 1.22 (s, 9H). |
| 193 | ¹H NMR (300 MHz, Chloroform-*d*) δ 7.65 - 7.54 (m, 1H), 7.31 (d, *J* = 8.4 Hz, 2H), 6.39 - 6.27 (m, 1H), 5.79 (s, 2H), 5.39 (d, *J =* 4.8 Hz, 2H), 3.96 (s, 3H), 3.87 - 3.76 (m, 6H). |
| 194 | ¹H NMR (300 MHz, Chloroform-*d*) δ 7.68 - 7.54 (m, 1H), 7.38 (d, *J* = 8.4 Hz, 1H), 5.44 (s, 2H), 4.04 - 3.93 (m, 9H). |
| 195 | ¹H NMR (300 MHz, Chloroform-*d*) δ 7.63 (dd, *J* = 8.4, 6.9 Hz, 1H), 7.41 - 7.35 (m, 1H), 5.44 (s, 2H), 4.55 - 4.41 (m, 2H), 400 (d, *J* = 1.2 Hz, 3H), 3.95 (s, 3H), 1.47 - 1.40 (m, 3H). |
| 196 | ¹H NMR (300 MHz, Chloroform-*d*) δ 7.69 - 7.59 (m, 1H), 7.44 - 7.33 (m, 1H), 6.34 - 5.90 (m, 1H), 5.50 (s, 2H), 4.68 - 4.52 (m, 2H), 4.01 (d, *J =* 3.0 Hz, 3H), 3.96 (s, 3H). |
| 197 | ¹H NMR (300 MHz, Chloroform-*d*) δ 7.62 (dd, *J* = 8.4, 6.9 Hz, 1H), 7.43 - 7.37 (m, 1H), 5.56 (s, 2H), 5.01 (s, 2H), 4.01 (d, *J =* 1.2 Hz, 3H), 3.99 (s, 3H). |
| 198 | ¹H NMR (300 MHz, Chloroform-*d*) δ 7.68 (dd, *J* = 8.4, 6.9 Hz, 1H), 7.42 - 7.35 (m, 1H), 5.48 (s, 2H), 4.05 - 3.96 (m, 6H), 2.15 - 2.11 (m, 6H). |
| 199 | ¹H NMR (300 MHz,DMSO-*d₆*) δ 7.62 - 7.58 (m, 1H), 7.51 - 7.48 (d, *J =* 7.8 Hz, 1H), 6.73 (s, 2H), 3.96 (s, 3H), 3.76 (s, 3H). |
| 200 | ¹H NMR (300 MHz, DMSO-*d*₆) δ 7.64 - 7.59 (m, 1H), 7.50 - 7.48 (d, *J* = 7.5 Hz, 1H), 6.73 (s, 2H), 3.95 (s, 3H), 3.75 (s, 3H). |
| 201 | ¹H NMR (300 MHz, Chloroform-*d*) δ 7.55 - 7.48 (m, 1H), 7.37 (d, *J =* 8.7 Hz, 1H), 5.60 (s, 2H), 4.00 (s, 3H), 3.99 - 3.94 (m, 3H), 3.96 - 3.94 (m, 3H). |
| 202 | ¹H NMR (300 MHz, Chloroform-*d*) δ 7.53 (t, *J* = 7.8 Hz, 1H), 7.35 - 7.30 (m, 1H), 5.54 (s, 2H), 4.46 (q, *J* = 7.2 Hz, 2H), 3.97 (s, 3H), 3.92 (s, 3H), 1.43 (t, *J =* 7.2 Hz, 3H). |
| 203 | ¹H NMR (300 MHz, Chloroform-*d*) δ 7.54 (t, *J* = 7.8 Hz, 1H), 7.43 - 7.31 (m, 1H), 5.50 (s, 2H), 4.36 (t, *J* = 6.6 Hz, 2H), 3.97 (s, 3H), 3.92 (s, 3H), 1.90 - 1.76 (m, 2H), 1.03 (t, *J* = 7.5 Hz, 3H). |
| 204 | ¹H NMR (300 MHz, Chloroform-*d*) δ 7.57 (t, *J =* 7.8 Hz, 1H), 7.41 - 7.31 (m, 1H), 5.48 - 5.20 (m, 3H), 3.97 (s, 3H), 3.92 (s, 3H), 1.42 (d, *J* = 6.3 Hz, 6H). |
| 205 | ¹H NMR (300 MHz, Chloroform-*d*) δ 7.52 (t, *J =* 7.8 Hz, 1H), 7.41 - 7.31 (m, 1H), 6.33 - 5.88 (m, 1H), 5.65 (s, 2H), 4.63 - 4.53 (m, 2H), 3.96 (s, 3H), 3.90 (s, 3H). |
| 209 | ¹H NMR (300 MHz, Chloroform-*d*) δ 7.52 (t, *J =* 7.8 Hz, 1H), 7.42 - 7.33 (m, 1H), 5.58 (s, 2H), 5.00 (s, 2H), 3.98 (s, 3H), 3.96 (s, 3H). |
| 210 | ¹H NMR (300 MHz, Chloroform-*d*) δ 7.54 (t, *J =* 7.8 Hz, 1H), 7.46 - 7.30 (m, 1H), 5.63 (s, 2H), 3.96 (s, 3H), 3.95 (s, 3H), 2.11 (s, 6H). |
| 212 | ¹H NMR (300 MHz, Chloroform-d) δ 7.51 (t, *J =* 7.8 Hz, 1H), 7.41 - 7.30 (m, 1H), 5.64 (s, 2H), 4.62 - 4.45 (m, 2H), 3.95 (s, 3H), 3.91 (s, 3H), 3.80 - 3.68 (m, 2H), 3.40 (s, 3H). |
| 214 | ¹H NMR (300 MHz, Chloroform-*d*) δ 7.51 - 7.47 (m, 1H), 7.38 - 7.31 (m, 1H), 5.64 (s, 2H), 4.55 (t, *J* = 6.9 Hz, 2H), 3.95 (s, 3H), 3.92 (s, 3H), 2.87 (t, *J* = 6.9 Hz, 2H), 2.19 (s, 3H). |
| 218 | ¹H NMR (300 MHz, Chloroform-*d*) δ 7.59 - 7.47 (m, 1H), 7.39 - 7.30 (m, 1H), 5.62 (s, 2H), 4.49 - 4.17 (m, 3H), 3.96 (s, 3H), 3.92 (s, 3H), 3.85 - 3.74 (m, 1H), 2.17 - 1.62 (m, 5H). |
| 219 | ¹H NMR (300 MHz, Chloroform-*d*) δ 7.51 - 7.37 (m, 3H), 7.35 - 7.23 (m, 4H), 5.44 (s, 2H), 5.37 (s, 2H), 3.90 (d, *J =* 0.9 Hz, 3H), 3.71 (d, *J =* 0.9 Hz, 3H). |
| 221 | ¹H NMR (300 MHz, DMSO-*d*₆) δ 7.67 - 7.48 (m, 4H), 3.88 (s, 3H), 3.75 (s, 3H), 2.31 (s, 3H). |
| 222 | ¹H NMR (300 MHz, Chloroform-*d*) δ 7.71 - 7.62 (m, 1H), 7.38 (dd, *J =* 8.4, 1.6 Hz, 1H), 5.59 (s, 2H), 3.99 (d, *J* = 0.9 Hz, 3H), 3.93 (d, *J* = 0.9 Hz, 3H), 3.00 (q, *J* = 7.2 Hz, 2H), 1.34 (t, *J* = 7.2 Hz, 3H) |
| 224 | ¹H NMR (300 MHz, Chloroform-*d*) δ 7.58 - 7.42 (m, 1H), 6.58 (d, *J* = 8.1 Hz, 1H), 5.41 (s, 2H), 3.98 (s, 3H), 3.96 (s, 3H), 3.93 (s, 3H), 2.30 - 2.18 (m, 1H), 1.05 - 0.98 (m, 2H), 0.76 - 0.67 (m, 2H). |
| 247 | ¹H NMR (300 MHz, CDCl₃) δ 7.91 (t, *J =* 7.8 Hz, 1H), 7.49 (d, *J =* 8.4 Hz, 1H), 6.86 - 6.36 (t, *J =* 74.1 Hz, 1H), 5.50 (s, 2H), 4.01 (s, 3H), 3.96 (s 3H). |

Several methods for preparing the compounds of the present invention are illustrated in detail in the following schemes and examples. Raw materials can be purchased on the market or can be prepared by methods known in literature or as shown in the detailed description. A person skilled in the art should understand that other synthetic routes may also be used to synthesize the compounds of the present invention. Although the specific raw materials and conditions in the synthetic routes have been illustrated below, they may be easily replaced with other similar raw materials and conditions. Various isomers and the like of the compounds resulted from these modifications or variants of the preparation methods of the present invention are all included in the scope of the present invention. In addition, the preparation methods as described below can be further modified according to the disclosure of the present invention using conventional chemical methods well known to a person skilled in the art. For example, the protection for appropriate groups during the reaction, etc.

Examples of methods provided below are used to enhance further understanding of the preparation methods of the present invention, and the specific substances, varieties and conditions employed are determined to be further illustrations of the present invention and not to limit its reasonable scope. Reagents used in the synthesis of the compounds shown in the following tables are either commercially available or can be easily prepared by a person of ordinary skill in the art.

Examples of representative compounds are as follows. The synthesis methods of other compounds are similar and will not be described in detail here.

### 1. Synthesis of Compound 3

(1) Compound 3-1 was added into 200 mL of THF, and n-BuLi (28 mL, 2.5 mol/L, 2.5 eq) was added dropwise into the reaction solution at -78°C under nitrogen protection. After adding, the solution was stirred for 1 h at this temperature, continued to be added dropwise with trimethyl borate (5.8 g, 2 eq) at -78°C, and then stirred at room temperature overnight. The LC-MS detection showed that the raw materials had almost been completely consumed, a major new peak was formed, and the MS signal was correct. The reaction solution was quenched using dilute hydrochloric acid. After the THF was removed by decompressing concentration, the reaction solution was extracted with ethyl acetate, washed with saturated saline solution (100 mL*3), and dried over anhydrous sodium sulfate, followed by isolation and purification through a silica gel column to obtain Compound 3-2 (white solid, 4.2 g, 54.4% yield). 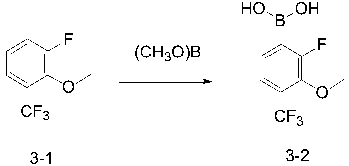
(2) At 25°C, Compound 3-2 (240 mg, 1 mmol, 1.5 eq) was dissolved in 10 mL of a mixed solvent (1,4-Dioxane : H₂O = 10:1) and added with cesium fluoride (315 mg. 3.0 eq), 6-amino-2-chloro-5-methoxypyrimidine-4-methyl carboxylate (Compound 3-3, 150 mg, 0.69 mmol, 1.0 eq), and Pd(dppf)Cl₂ (28 mg, 0.05 eq). The reaction solution was heated up to 100°C under nitrogen protection and stirred for 12 h. The reaction was monitored by LC-MS until its completion. The solvent was removed by concentration. The solution was stirred with silica gel and purified by normal phase chromatography to obtain Compound 3 (white solid, 60 mg, 25.5% yield).

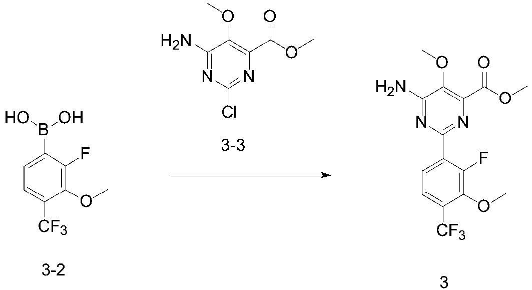

### 2. Synthesis of Compound 24

At 25°C, the DCM solution of Compound 24-1 (100 mg, 0.28 mol, 1.0 eq) was added with triethylamine (3.0 eq), ethanol (20 mg, 1.5 eq) in slow drops, and finally PyBOP (215 mg, 1.5 eq) and reacted for 1 h. When the reaction was complete, the reaction solution was poured into water to quench the reaction and extracted with DCM for three times. The organic phases were combined, washed once with saturated saline solution, dried over anhydrous sodium sulfate, concentrated and then stirred, followed by isolation and purification through a silica gel column to obtain Compound 24 (52 mg, white solid).

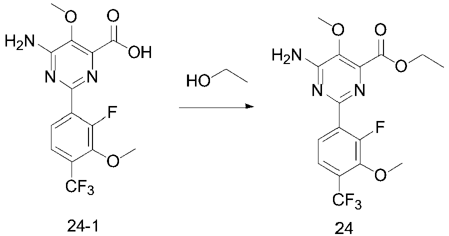

### 3. Synthesis of Compound 62

At 25°C, the DCM solution of Compound 24-1 (100 mg, 0.28 mol, 1.0 eq) was added with triethylamine (3.0 eq), trifluoroethanol (42 mg, 1.5 eq) in slow drops, and finally PyBOP (215 mg, 1.5 eq) and reacted for 1 h. When the reaction was complete, the reaction solution was poured into water to quench the reaction and extracted with DCM for three times. The organic phases were combined, washed once with saturated saline solution, dried over anhydrous sodium sulfate, concentrated and then stirred, followed by isolation and purification through a silica gel column to obtain Compound 62 (30 mg, white solid).

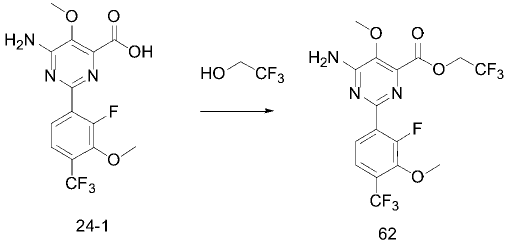

### 4. Synthesis of Compound 74

(1) The Compound 3 (50 g, 1.0 eq) was dissolved in 500 mL of THF/H₂O (v/v = 4:1) and at 0°C, added with LiOH·H₂O (14 g, 2.5 eq). The reaction solution was reacted for 2 h at 0°C. LC-MS detected disappearance of the raw materials and the main product peak. After THF being removed by concentration, the solution was adjusted to pH = 2-3 using 4N hydrochloric acid and solids precipitated after cooling which were subsequently filtered and dried to obtain the white solid 24-1 (43 g, 89% yield). 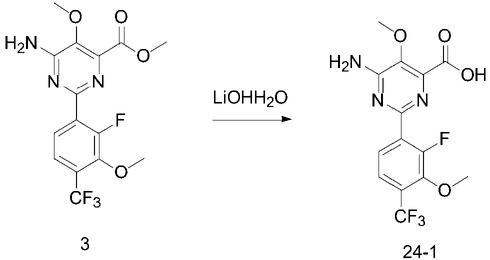
(2) Compound 24-1 (23 g, 1.0 eq) was dissolved in 250 mL of DCM. The solution was added with triethylamine (12.9 g, 2.0 eq) and ethylene glycol methyl ether (9.7 g, 2.0 eq). At 0°C, PyBOP (39.7 g, 1.2 eq) was added in batches, then heated up to room temperature and reacted for 1 hour. When the main product peak was detected by LC-MS, the reaction solution was poured into water to quench the reaction, extracted with DCM for three times. The organic phases were combined, washed once with saline solution and concentrated, followed by isolation and purification by column chromatography to obtain white the solid 74 (22 g, 82% yield).

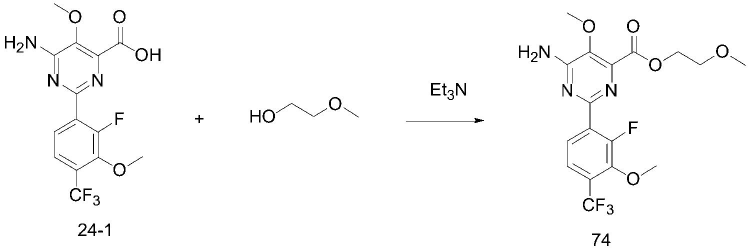

Or, Compound 3 (5 g, 13.3 mmol) was added into a three-necked flask containing ethylene glycol methyl ether (20.0 g) at normal temperature, added with tetraisopropyl titanate (0.1 g, 0.35 mmol), heated up to 120-125°C, and reacted for 1.5-2 h. When LC detected the completion of the reaction, ethylene glycol monomethyl ether was removed by decompressing concentration, and the solution was isolated and purified through a silica gel column to obtain 5.0 g of the white solid Compound 74 (11.9 mmol, 89.5% yield).

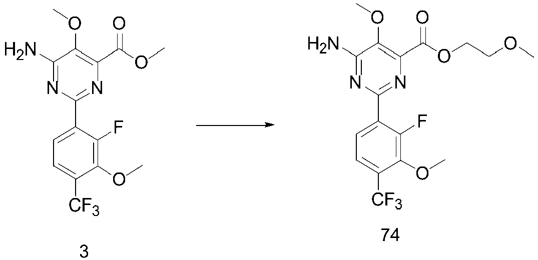

### 5. Synthesis of Compound 106

At 25°C, the DCM solution of Compound 24-1 (100 mg, 0.28 mol, 1.0 eq) was added with triethylamine (3.0 eq), trifluoroethanol (30 mg, 1.5 eq) in slow drops, and finally PyBOP (215 mg, 1.5 eq) and reacted for 1 h. When the reaction was complete, the reaction solution was poured into water to quench the reaction and extracted with DCM for three times. The organic phases were combined, washed once with saturated saline solution, dried over anhydrous sodium sulfate, concentrated and then stirred, followed by isolation and purification through a silica gel column to obtain Compound 106 (50 mg, white solid).

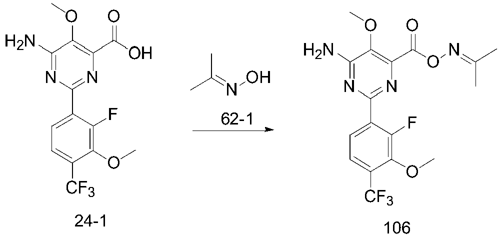

### 6. Synthesis of Compound 116

At 25°C, the DCM solution of Compound 24-1 (100 mg, 0.2 mol, 1.0 eq) was added with triethylamine (3.0 eq), cyclopropanol (25 mg, 1.5 eq) in slow drops, and finally PyBOP (215 mg, 1.5 eq) and reacted for 1 h. When the reaction was complete, the reaction solution was poured into water to quench the reaction and extracted with DCM for three times. The organic phases were combined, washed once with saturated saline solution, dried over anhydrous sodium sulfate, concentrated, and then stirred and passed through a column to obtain Compound 116 (31 mg, white solid).

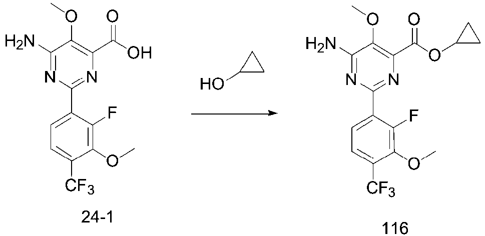

### 7. Synthesis of Compound 201 and 221

Compound 201-1 (19 g, 0.1 mol, 1 eq), Et₃N (30.6 g, 0.3 mol, 3 eq) and S-methylisothiourea sulfate (18.8 g, 0.1 mol, 1 eq) were dissolved with methanol (200 mL) in a 500 mL single-necked flask and refluxed at 80°C for 12 h. When the completion of the reaction was detected, the reaction solution was spin-dried, extracted and separated using water and ethyl acetate. The organic phase was washed twice with saturated saline solution, dried over anhydrous sodium sulfate, stirred, and isolated by normal phase chromatography to obtain Compound 201-2 (17 g, 73.9% yield).

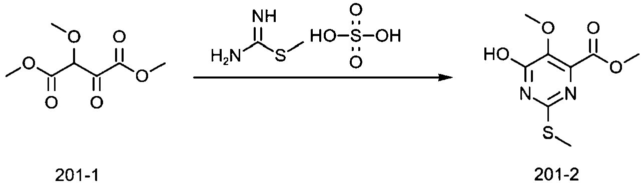

The crude product 201-2 (17 g, 73 mmol, 1 eq) obtained above and N,N-diethyl aniline (5.5 g, 36.9 mmol, 0.5 eq) were dissolved with phosphorus oxychloride (200 mL) in a 500 mL single-necked flask and refluxed at 80°C for 12 h. When the completion of the reaction was detected, the reaction solution was spin-dried, extracted and separated using water and ethyl acetate. The organic phase was washed twice with saturated saline solution, dried over anhydrous sodium sulfate, stirred, and isolated by normal phase chromatography to obtain Compound 201-3 (14 g, 77.8% yield).

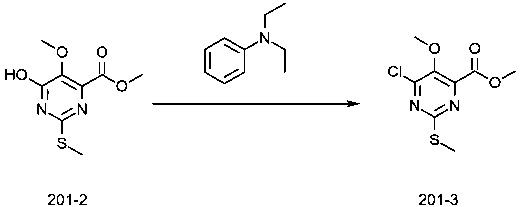

The crude product 201-3 (14 g, 5.6 mmol, 1 eq) obtained above and ammonium carbonate (27 g, 282 mmol, 5 eq) were dissolved with DMSO (200 mL) in a 500 mL single-necked flask and refluxed at 50°C for 12 h. When the completion of the reaction was detected, the reaction solution was extracted and separated using water and ethyl acetate. The organic phase was washed twice with saturated saline solution, dried over anhydrous sodium sulfate, stirred, and isolated by normal phase chromatography to obtain Compound 201-4 (9.6 g, 74.4% yield).

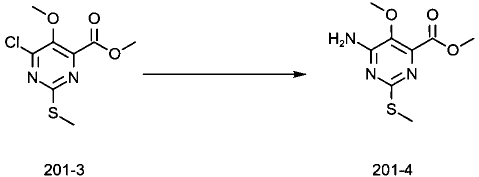

Compound 201-5 (2.1 g, 8.5 mmol, 1 eq), Compound 201-4 (2.14 g, 9.35 mmol, 1.1 eq), copper(I) thiophene-2-carboxylate (4.8 g, 25.5 mmol, 3 eq) and trifurylphosphine (0.39 g, 1.7 mmol, 0.2 eq) were dissolved with DMF (40 mL) in a 250 mL single-necked flask, replaced once by nitrogen gas, and added with Pd(dba)₃ (80.8 mg, 0.085 mmol, 0.01 eq). Next, nitrogen gas replacement was performed for three times. The reaction solution was refluxed at 100°C for 12 h. When the completion of the reaction was detected, the reaction solution was extracted and separated using water and ethyl acetate. The organic phase was washed twice with saturated saline solution, then dried over anhydrous sodium sulfate, stirred, and isolated by normal phase chromatography (EA/PE = 1/8) to obtain Compound 201 (691 mg, 21.6% yield).

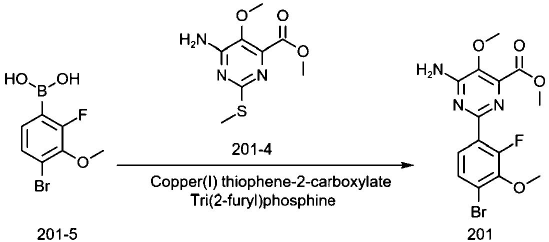

Compound 201 (691 mg, 1.8 mmol, 1 eq) was dissolved in a mixture of tetrahydrofuran (16 mL) and water (4 mL) in a 100 mL single-necked flask, added with lithium hydroxide (0.22 g, 8.9 mmol, 5 eq) while being stirred at room temperature, and reacted at room temperature for 4 h. When the completion of the reaction was detected, the tetrahydrofuran was spin-dried. Diluted hydrochloric acid was added to adjust the solution to acidity, and the product acid precipitated which was then suction-filtered to obtain Compound 221-1 (589 mg, 88.7% yield).

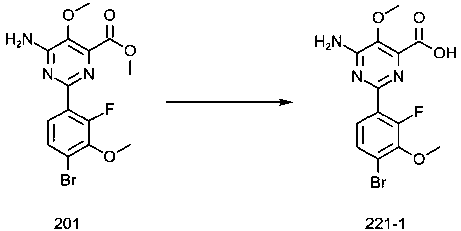

Compound 221-1 (0.1 g, 0.27 mmol, 1 eq), methyl mercaptan (25.9 mg, 0.54 mmol. 2 eq) and triethylamine (81.8 mg, 0.81 mmol, 3 eq) were dissolved with 20 mL of dichloromethane in a 50 mL single-necked flask, added with PyBOP (168.8 mg, 0.32 mmol, 1.2 eq) while being stirred at room temperature, and reacted for 1 h. When the completion of the reaction was detected, the solution was immediately added with silica gel and stirred, and isolated by normal phase chromatography (EA/PE = 1/10) to obtain Compound 221 (79 mg, 73.2% yield).

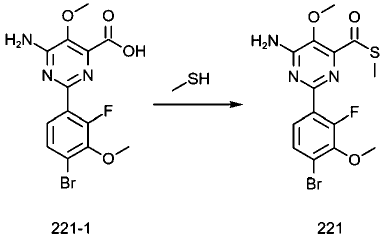

### 8. Synthesis of Compound 224

Compound 224-1 (0.341 g, 1 mmol, 1 eq) was dissolved in 20 mL of toluene and 1 mL of water, added with cyclopropylboric acid (0.103 g, 1.2 mmol, 1.2 eq) and potassium phosphate (0.742 g, 3.5 mmol, 3.5 eq), replaced with N₂, added with palladium acetate (8.3 mg, 0.05 mmol, 0.05 eq) and tricyclohexylphosphine (28.1 mg, 0.1 mmol, 0.1 eq), replaced with N₂ for 3 times, heated up to 80°C, and reacted for 12 h. The reaction was monitored until the starting materials had disappeared. After being spin-dried, the solution was added with water, extracted three times with ethyl acetate, washed three times with saturated saline solution, dried over anhydrous sodium sulfate, and then purified through a column. The fraction was spin-dried to obtain Compound 224 (139 mg, 40.1% yield).

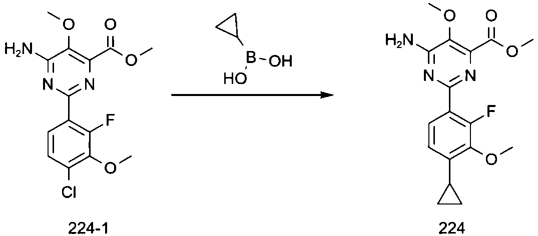

### Evaluation of Biological Activity:

The criteria for activity levels of plant damage (i.e., growth control rate) are as follows:
Level 9: completely dead;
Level 8: growth control rate is greater than or equal to 90% and less than 100%;
Level 7: growth control rate is greater than or equal to 80% and less than 90%;
Level 6: growth control rate is greater than or equal to 70% and less than 80%;
Level 5: growth control rate is greater than or equal to 50% and less than 70%;
Level 4: growth control rate is greater than or equal to 30% and less than 50%;
Level 3: growth control rate is greater than or equal to 20% and less than 30%;
Level 2: growth control rate is greater than or equal to 10% and less than 20%;
Level 1: growth control rate is less than 10%;
Level 0: no effect.

The above growth control rates are fresh weight control rates.

### Post-emergence test experiment:

Monocotyledonous and dicotyledonous weed seeds (*Descurainia sophia, Capsella bursa-pastoris, Abutilon theophrasti, Galium spurium, Stellaria media, Lithospermum arvense, Rorippa indica, Alopecurus aequalis, Alopecurus japonicus, Eleusine indica, Beckmannia syzigachne, Sclerochloa dura, Erigeron canadensis, Phleum paniculatum, Veronica, Bromus japonicus, Aegilops tauschii, Phalaris arundinacea, Amaranthus retroflexus, Chenopodium, Commelina communis, Cichorium endivia, Convolvulus arvensis, Cirsium arvense, Solanum nigrum, Acalypha australis, Digitaria sanguinalis, Echinochloa crusgalli, Setaria viridis, Setaria pumila, Leptochloa chinensis, Monochoria vaginalis, Sagittaria trifolia, Schoenoplectiella juncoides, Cyperus rotundus, Cyperus iria, Cyperus difformis, Fimbristylis, Portulaca oleracea, Xanthium sibiricum, Ipomoea nil, Eschenbachia japonica,* etc.) and major crop seeds (wheat, maize, paddy rice, soybean, cotton, oilseed rape, millet, sorghum, potato, sesame, castor plant, Chinese cabbage, radish, etc.) were placed in plastic pots filled with soil and then covered with 0.5-2 cm of soil to allow them to grow in a favorable greenhouse environment. Two weeks after sowing, the test plants were treated at the stage of 2-3 leaves. The test compounds of the present invention were individually dissolved with acetone, then added with Tween^{®} 80, with 1.5 L/ha of methyl oleate emulsifiable concentrate as a synergist, diluted into solution of a certain concentration using a certain amount of water, and sprayed to plants using a spray tower. After drug application, the experimental results on weeds were collected after 3 weeks of cultivation in a greenhouse. The compounds were applied at 120, 60, 30, and 15 g a.i./ha in triplicate, and the results were averaged. Representative data were listed in Table 3.

**Table 3 Results of post-emergence test on weeds**

| **No.** | ***Veronica*** | **Maize** | **Wheat** | **Paddy rice** | **Oilseed rape** | **Chinese cabbage** | **Radish** | **Dosage (g a.i./ha)** |
|---|---|---|---|---|---|---|---|---|
| 1 | 9 | 0 | 0 | 0 | 0 | 0 | 0 | 30 |
| 2 | 9 | 0 | 0 | 0 | 0 | 0 | 0 | 30 |
| 3 | 9 | 0 | 0 | 0 | 0 | 0 | 0 | 30 |
| 4 | 9 | 0 | 0 | 0 | 0 | 0 | 0 | 30 |
| 21 | 9 | 0 | 0 | 0 | 0 | 0 | 0 | 30 |
| 22 | 9 | 0 | 0 | 0 | 0 | 0 | 0 | 30 |
| 23 | 9 | 0 | 0 | 0 | 0 | 0 | 0 | 30 |
| 24 | 9 | 0 | 0 | 0 | 0 | 0 | 0 | 30 |
| 25 | 9 | 0 | 0 | 0 | 0 | 0 | 0 | 30 |
| 26 | 9 | 0 | 0 | 0 | 0 | 0 | 0 | 30 |
| 52 | 9 | 0 | 0 | 0 | 0 | 0 | 0 | 30 |
| 57 | 9 | 0 | 0 | 0 | 0 | 0 | 0 | 30 |
| 58 | 9 | 0 | 0 | 0 | 0 | 0 | 0 | 30 |
| 59 | 9 | 0 | 0 | 0 | 0 | 0 | 0 | 30 |
| 60 | 9 | 0 | 0 | 0 | 0 | 0 | 0 | 30 |
| 61 | 9 | 0 | 0 | 0 | 0 | 0 | 0 | 30 |
| 62 | 9 | 0 | 0 | 0 | 0 | 0 | 0 | 30 |
| 63 | 9 | 0 | 0 | 0 | 0 | 0 | 0 | 30 |
| 64 | 9 | 0 | 0 | 0 | 0 | 0 | 0 | 30 |
| 72 | 9 | 0 | 0 | 0 | 0 | 0 | 0 | 30 |
| 73 | 9 | 0 | 0 | 0 | 0 | 0 | 0 | 30 |
| 74 | 9 | 0 | 0 | 0 | 0 | 0 | 0 | 30 |
| 76 | 9 | 0 | 0 | 0 | 0 | 0 | 0 | 30 |
| 81 | 9 | 0 | 0 | 0 | 0 | 0 | 0 | 30 |
| 82 | 9 | 0 | 0 | 0 | 0 | 0 | 0 | 30 |
| 93 | 9 | 0 | 0 | 0 | 0 | 0 | 0 | 30 |
| 100 | 9 | 0 | 0 | 0 | 0 | 0 | 0 | 30 |
| 103 | 9 | 0 | 0 | 0 | 0 | 0 | 0 | 30 |
| 106 | 9 | 0 | 0 | 0 | 0 | 0 | 0 | 30 |
| 116 | 9 | 0 | 0 | 0 | 0 | 0 | 0 | 30 |
| 117 | 9 | 0 | 0 | 0 | 0 | 0 | 0 | 30 |
| 119 | 9 | 0 | 0 | 0 | 0 | 0 | 0 | 30 |
| 121 | 9 | 0 | 0 | 0 | 0 | 0 | 0 | 30 |
| 129 | 9 | 0 | 0 | 0 | 0 | 0 | 0 | 30 |
| 130 | 9 | 0 | 0 | 0 | 0 | 0 | 0 | 30 |
| 133 | 9 | 0 | 0 | 0 | 0 | 0 | 0 | 30 |
| 150 | 9 | 0 | 0 | 0 | 0 | 0 | 0 | 30 |
| 151 | 9 | 0 | 0 | 0 | 0 | 0 | 0 | 30 |
| 152 | 9 | 0 | 0 | 0 | 0 | 0 | 0 | 30 |
| 155 | 9 | 0 | 0 | 0 | 0 | 0 | 0 | 30 |
| 156 | 9 | 0 | 0 | 0 | 0 | 0 | 0 | 30 |
| 157 | 9 | 0 | 0 | 0 | 0 | 0 | 0 | 30 |
| 190 | 9 | 0 | 0 | 0 | 0 | 0 | 0 | 30 |
| 191 | 9 | 0 | 0 | 0 | 0 | 0 | 0 | 30 |
| 192 | 9 | 0 | 0 | 0 | 0 | 0 | 0 | 30 |
| 193 | 9 | 0 | 0 | 0 | 0 | 0 | 0 | 30 |
| 199 | 9 | N | 1 | 1 | N | N | N | 30 |
| 200 | 9 | N | 1 | 1 | N | N | N | 30 |
| 201 | 9 | N | 1 | 1 | N | N | N | 30 |
| 202 | 9 | N | 1 | 1 | N | N | N | 30 |
| 203 | 9 | N | 1 | 1 | N | N | N | 30 |
| 204 | 9 | N | 1 | 1 | N | N | N | 30 |
| 205 | 9 | N | 1 | 1 | N | N | N | 30 |
| 209 | 9 | N | 0 | 1 | N | N | N | 30 |
| 210 | 9 | N | 1 | 1 | N | N | N | 30 |
| 212 | 9 | N | 1 | 1 | N | N | N | 30 |
| 214 | 9 | N | 1 | 1 | N | N | N | 30 |
| 218 | 9 | 2 | 0 | 0 | N | N | N | 30 |
| 219 | 9 | 2 | 0 | 0 | N | N | N | 30 |
| 221 | 9 | 1 | 0 | 0 | 1 | 1 | 1 | 30 |
| 222 | 9 | 2 | 0 | 0 | 1 | 1 | 2 | 30 |
| 247 | 9 | 0 | 0 | 0 | 0 | 0 | 0 | 30 |
| Control compound A | N | 4 | 3 | 3 | 5 | 5 | 5 | 30 |

Note: N represents untested; Control compound A:

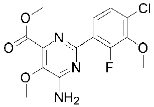

### Pre-emergence test experiment:

Monocotyledonous and dicotyledonous weed seeds as well as major crop seeds (wheat, maize, paddy rice, soybean, cotton, oilseed rape, millet, and sorghum) were placed in plastic pots filled with soil and then covered with 0.5-2 cm of soil. The test compounds of the present invention were individually dissolved with acetone, then added with Tween^{®} 80, diluted with a certain amount of water to obtain a solution of a certain concentration, and sprayed immediately after sowing. After drug application, the experimental results were observed after 4 weeks of cultivation in a greenhouse, showing that most of the drugs of the present invention exhibited outstanding effects at the dose of 250 g a.i./ha., especially on weeds such as *Echinochloa crusgalli, Digitaria sanguinalis, Abutilon theophrasti,* etc., and many of the compounds had great selectivity to maize, wheat, paddy rice, and soybean.

Meanwhile, it has been found by experimentations on major weeds in wheat fields and paddy rice fields that the compounds of the present invention generally have good weed control effects; in particular, it has been noticed that the compounds have extremely high activity on broad-leaf weeds and *Cyperaceae* that are resistant to ALS inhibitors, for example, *Sagittaria trifolia, Schoenoplectiella juncoides, Cyperus difformis, Descurainia sophia, Capsella bursa-pastoris, Lithospermum arvense, Galium spurium, Cyperus rotundus,* etc., which have excellent commercial values.

### Safety evaluation on transplanted paddy rice and control effect evaluation on weeds in paddy fields:

After paddy field soil was loaded into a 1/1,000,000 ha pot, the seeds of *Monochoria vaginalis, Pontederia korsakowii* and *Bidens tripartita* were sowed and gently covered with soil, then left to stand in a greenhouse with water storage of 0.5-1 cm deep. The tuber of *Sagittaria trifolia* was planted on the next day or 2 days later. The water storage was kept at a depth of 3-4 cm thereafter. When the *Monochoria vaginalis, Pontederia korsakowii* and *Bidens tripartita* reached their 0.5-leaf stages and the *Sagittaria trifolia* reached the primary leaf stage, WP or SC aqueous dilutions of the compounds of the present invention formulated according to customary formulation methods were homogeneously dripped for treatment to the specified effective amount using pipettes.

In addition, after being loaded into the 1/1,000,000 ha pot, the paddy field soil was leveled to keep the water storage at a depth of 3-4 cm. The paddy rice (japonica rice) at 3-leaf stage was transplanted at a transplanting depth of 3 cm on the next day. The compounds of the present invention were treated in the same manner as above on the 5^{th} day after the transplantation.

The developmental conditions of *Monochoria vaginalis, Pontederia korsakowii, Bidens tripartita* and *Sagittaria trifolia* on the 14^{th} day after the drug treatment and the developmental conditions of paddy rice on the 21^{st} day after the drug treatment were observed with the naked eye, respectively. The effects were evaluated according to the above-mentioned criteria for activity levels. Many compounds exhibited excellent activity and selectivity, and the representative results are as shown in Table 4.

**Table 4 Safety evaluation on transplanted paddy rice and control effect evaluation on weeds in paddy fields**

| **No.** | ***Monochoria vaginalis*** | ***Pontederia korsakowii*** | ***Bidens tripartita*** | **Paddy rice** | **Dosage (g a.i./ha)** |
|---|---|---|---|---|---|
| 2 | 9 | 9 | 9 | 0 | 75 |
| 3 | 9 | 9 | 9 | 0 | 75 |
| 4 | 9 | 9 | 9 | 0 | 75 |
| 24 | 9 | 9 | 9 | 0 | 75 |
| 62 | 9 | 9 | 9 | 0 | 75 |
| 106 | 9 | 9 | 9 | 0 | 75 |
| 116 | 9 | 9 | 9 | 0 | 75 |
| pyrazosulfuron-ethyl | 1 | 2 | 4 | 1 | 30 |

| | | | | | |
|---|---|---|---|---|---|
| Note: *Monochoria vaginalis, Pontederia korsakowii* and *Bidens tripartita* seeds were collected from Heilongjiang, China and tested to be resistant to conventional doses of pyrazosulfuron-ethyl. | | | | | |

### Activity assay of compositions:

The required active ingredient B was purchased from a reagent company or a technical material manufacturer or synthesized by a conventional method. The active ingredient A was Compound 74 in Table 1. The technical materials were all dissolved with acetone solvents and diluted with aqueous solution of 0.1% Tween^{®} 80 emulsifier, which should be used immediately after dilution.

### (A) Post-emergence spray treatment of stems and leaves:

Weeds were cultivated by a pot culture method. A 180×140 mm plastic nutritional bowl, containing air-dried and sieved topsoil (taking up 4/5 of the bowl) collected from the field, was placed in an enamel pan, wherein the soil had an initial moisture content of 20%. Weed seeds with plump and uniform grains were selected, soaked in lukewarm water at 25°C for 6 hours, and accelerated to germinate in a 28°C biochemical incubator (in darkness). The weed seeds that had just germinated were evenly placed on the surface of the soil and then covered with 0.5-1 cm soil according to the sizes of seeds.

The weeds were cultured in a controllable sunlight greenhouse at 20-30°C, in natural light, and with relative humidity of 57%-72%. The soil was loam with an organic matter content of 1.63%, a pH value of 7.1, alkali-hydrolyzable nitrogen of 84.3 mg/kg, rapidly available phosphorus of 38.5 mg/kg, and rapidly available potassium of 82.1 mg/kg.

Each treatment was repeated 4 times for 3 pots. Each pot was seeded with 20 weed seeds.

The drugs were applied once in total in the experiment. When at the stage of 1.5-2 leaves, the weeds were thinned out to maintain 10 strains per pot. Thirty strains were kept in each treatment and then continued to be cultured until the *Erigeron canadensis* reached 10 cm tall and other weeds in their 3-4 leaf stage for further treatment.

The well-cultured test materials were evenly placed on a 0.5 m² platform and foliage-sprayed by the 3WP-2000-type walking spray tower at a dosage of 450 kg/ha and a spray pressure of 0.3 MPa. After all the drug solution was sprayed, the air valve was closed. And 30 seconds later, the door of the spray tower was opened, and the nutritional bowl was taken out. Next, the air valve was opened, and the spray tube was cleaned by spraying 50 ml of water. The test materials were transferred to and routinely cultured in a greenhouse after the treatment.

### (B) Data investigation and statistical analysis

A method for investigating absolute numbers was employed. The whole seedlings of the survival weeds were cut along the soil surface with a blade, and the fresh weight of the weeds was weighed by an analytical balance. For dead weeds, the fresh weight thereof was considered zero.

The investigation was performed 21 days after the treatment for once in total.

The theoretical fresh weight inhibition rate of a mixed combination of each treatment was calculated by the Gowing method (E0 = X + Y - X * Y / 100). Then compared with the measured inhibition rate (E), the type of the combined action of the combination of the two on weeds was evaluated, which was a synergistic effect when E - E0 > 10%; an antagonistic effect when E - E0 < -10%; an additional effect when -10% ≤ E - E0 ≤ 10%. An optimal ratio was determined according to factors such as the actual control effects, characteristics of herbicides, balance of the formulation, etc. In the formula, X represents the fresh weight inhibition rate of the active ingredient A in a dosage of P; Y represents the fresh weight inhibition rate of the active ingredient B in a dosage of Q. The statistical results are shown in Table 5 below.

**Table 5 Evaluation of actual control effect and combined action on weeds of a combination of A and B**

| **Component (A + B)** | **Weed** | **Dose of A + B (g a.i./ha)** | **A:B** | **Control effect of A applied alone in a corresponding dose (%)** | **Control effect of B applied alone in a corresponding dose (%)** | **Control effect of A + B (%), E** | **E0 (%)** | **E - E0 (%)** |
|---|---|---|---|---|---|---|---|---|
| A + Bipyrazone | *Euphorbia helioscopia* | 3 + 7.5 | 2:5 | 58.0 | 37.1 | 85.2 | 73.6 | 11.6 |
| | | 3 + 15 | 1:5 | 58.0 | 55.8 | 94.7 | 81.4 | 13.3 |
| A + Fenpyrazone | *Euphorbia helioscopia* | 3 + 15 | 1:5 | 58.0 | 34.3 | 91.4 | 72.4 | 19.0 |
| | | 3 + 30 | 1:10 | 58.0 | 60.0 | 100.0 | 83.2 | 16.8 |
| 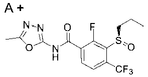 | *Euphorbia helioscopia* | 3 + 15 | 1:5 | 58.0 | 34.3 | 86.6 | 72.4 | 14.2 |
| | | 3 + 30 | 1:10 | 58.0 | 55.4 | 97.7 | 81.3 | 16.4 |
| A + Tripyrasulfone | *Euphorbia helioscopia* | 3 + 15 | 1:5 | 58.0 | 20.0 | 77.5 | 66.4 | 11.1 |
| | | 3 + 30 | 1:10 | 58.0 | 36.9 | 87.4 | 73.5 | 13.9 |
| A + Cypyrafluone | *Euphorbia helioscopia* | 3 + 15 | 1:5 | 58.0 | 34.7 | 83.3 | 72.6 | 10.7 |
| | | 3 + 30 | 1:10 | 58.0 | 55.2 | 94.0 | 81.2 | 12.8 |
| A + Fluroxypyr-meptyl | *Veronica* | 3 + 60 | 1:20 | 62.3 | 20.3 | 82.5 | 70.0 | 12.5 |
| | | 3 + 120 | 1:40 | 62.3 | 40.6 | 90.6 | 77.6 | 13.0 |
| A + Halauxifen-methyl | *Veronica* | 3 + 1.5 | 2:1 | 62.3 | 22.8 | 82.3 | 70.9 | 11.4 |
| | | 3 + 3 | 1:1 | 62.3 | 43.1 | 93.4 | 78.5 | 14.9 |
| A + Florpyrauxifen-benzyl | *Veronica* | 3 + 3 | 1:1 | 62.3 | 20.1 | 81.7 | 69.9 | 11.8 |
| | | 3 + 6 | 1:2 | 62.3 | 45.8 | 95.0 | 79.6 | 15.4 |
| A + Benazolin-ethyl | *Veronica* | 3 + 75 | 1:25 | 62.3 | 24.0 | 82.6 | 71.3 | 11.3 |
| | | 3 + 150 | 1:50 | 62.3 | 35.2 | 90.0 | 75.6 | 14.4 |
| A + Clopyralid | *Veronica* | 3 + 45 | 1:15 | 62.3 | 12.4 | 79.0 | 67.0 | 12.0 |
| | | 3 + 90 | 1:30 | 62.3 | 33.3 | 88.4 | 74.9 | 13.5 |
| A + Aminopyralid | *Veronica* | 3 + 30 | 1:10 | 62.3 | 18.0 | 83.7 | 69.1 | 14.6 |
| | | 3 + 60 | 1:20 | 62.3 | 39.7 | 96.5 | 77.3 | 19.2 |
| A + Picloram | *Veronica* | 3 + 15 | 1:5 | 62.3 | 15.7 | 80.5 | 68.2 | 12.3 |
| | | 3 + 30 | 1:10 | 62.3 | 30.8 | 86.0 | 73.9 | 12.1 |
| A + MCPA-isooctyl | *Veronica* | 3 + 150 | 1:50 | 62.3 | 23.5 | 86.0 | 71.2 | 14.8 |
| | | 3 + 300 | 1:100 | 62.3 | 40.8 | 94.8 | 77.7 | 17.1 |
| A + Florasulam | *Veronica* | 3 + 3 | 1:1 | 62.3 | 15.7 | 80.0 | 68.2 | 11.8 |
| | | 3 + 6 | 1:2 | 62.3 | 28.1 | 85.9 | 72.9 | 13.0 |
| A + Bentazone | *Monochoria* | 3 + 150 | 1:50 | 66.4 | 45.2 | 94.3 | 81.6 | 12.7 |
| | *vaginalis* | 3 + 300 | 1:100 | 66.4 | 60.5 | 99.7 | 86.7 | 13.0 |
| A + Propanil | *Monochoria vaginalis* | 3 + 600 | 1:200 | 66.4 | 22.1 | 85.0 | 73.8 | 11.2 |
| | | 3 + 1200 | 1:400 | 66.4 | 37.4 | 90.4 | 79.0 | 11.4 |
| 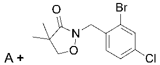 | *Euphorbia helioscopia* | 3 + 60 | 1:20 | 58.0 | 25.7 | 82.3 | 68.8 | 13.5 |
| | | 3 + 120 | 1:40 | 58.0 | 44.6 | 90.5 | 76.7 | 13.8 |
| 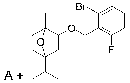 | *Lindernia procumbens* | 3 + 7.5 | 2:5 | 37.7 | 42.8 | 76.4 | 64.4 | 12.0 |
| | | 3 + 15 | 1:5 | 37.7 | 60.7 | 88.4 | 75.5 | 12.9 |

Meanwhile, it has been found by extensive experimentations that many of the compounds of the present invention and compositions thereof have good selectivity to gramineae grasses such as zoysia grass, bermuda grass, tall fescue, bluegrass, ryegrass, seashore paspalum, etc., and can prevent and control many key gramineous weeds and broad-leaf weeds. The experiments on sugarcane, soybean, cotton, oil sunflower, potato, fruit trees, vegetables and the like using different herbicide application methods also demonstrated excellent selectivity and commercial values.

## Claims

1. A benzene ring-substituted pyrimidinecarboxylic acid compound, as shown in the general formula I: 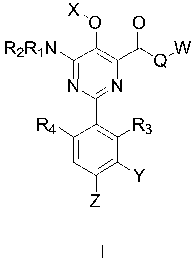 wherein,
X represents unsubstituted or halogen-substituted alkyl;
Y represents OR₅;
Z represents bromine, haloalkyl, or cycloalkyl;
Q represents O or S;
R₁ and R₂ each independently represent hydrogen;
R₃ represents halogen or amino;
R₄ represents hydrogen or halogen;
R₅ represents unsubstituted or halogen-substituted alkyl;
W represents hydrogen or its salt, alkyl, alkenyl, alkynyl, cycloalkyl, cycloalkenyl, heterocyclyl, aryl, 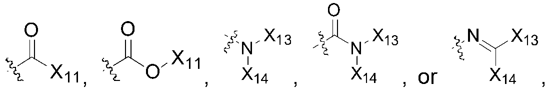 wherein, the "alkyl", "alkenyl" or "alkynyl" is optionally substituted by at least one group selected from halogen, cyano, nitro, cycloalkyl, trialkylsilyl, cycloalkenyl, heterocyclyl, aryl, 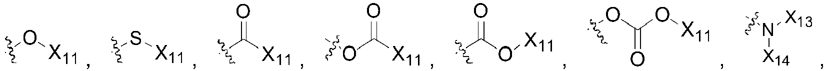 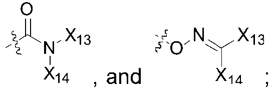 the "cycloalkyl", "cycloalkenyl", "heterocyclyl" or "aryl" is optionally substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₀, -SR₁₀, -(CO)OR₁₀, -(SO₂)R₁₀, -N(R₁₀)₂ and -O-alkylene-(CO)OR₁₀, or two adjacent carbon atoms on the ring form a fused ring with - OCH₂CH₂- or -OCH₂O- that is unsubstituted or substituted by halogen;
X₁₁ each independently represents hydrogen, alkyl, alkenyl, alkynyl, cycloalkyl, cycloalkylalkyl, cycloalkenyl, cycloalkenylalkyl, heterocyclyl, heterocyclylalkyl, aryl, or arylalkyl, wherein, the "alkyl", "alkenyl" or "alkynyl" is optionally substituted by at least one group selected from halogen and alkoxy; the "cycloalkyl", "cycloalkylalkyl", "cycloalkenyl", "cycloalkenylalkyl", "heterocyclyl", "heterocyclylalkyl", "aryl" or "arylalkyl" is optionally substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₀, -SR₁₀, -(CO)OR₁₀, -(SO₂)R₁₀, -N(R₁₀)₂ and -O-alkylene-(CO)OR₁₀, or two adjacent carbon atoms on the ring form a fused ring with - OCH₂CH₂- or -OCH₂O- that is unsubstituted or substituted by halogen;
X₁₃ and X₁₄ each independently represent hydrogen, halogen, cyano, alkoxy, alkoxyalkyl, alkylcarbonyl, alkoxycarbonyl, alkylsulfonyl, alkyl, alkenyl, alkynyl, cycloalkyl, cycloalkylalkyl, cycloalkenyl, cycloalkenylalkyl, aryl, arylalkyl, heterocyclyl or heterocyclylalkyl, or the CX₁₃X₁₄ group together forms an unsubstituted or substituted cyclic structure, or the NX₁₃X₁₄ group together forms an unsubstituted or substituted heterocyclyl with a nitrogen atom at 1-position, wherein, the "alkyl", "alkenyl" or "alkynyl" is optionally substituted by halogen; the "cycloalkyl", "cycloalkylalkyl", "cycloalkenyl", "cycloalkenylalkyl", "aryl", "arylalkyl", "heterocyclyl" or "heterocyclylalkyl" is optionally substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₀, -SR₁₀, -(CO)OR₁₀, - (SO₂)R₁₀, -N(R₁₀)₂ and -O-alkylene-(CO)OR₁₀, or two adjacent carbon atoms on the ring form a fused ring with -OCH₂CH₂- or -OCH₂O- that is unsubstituted or substituted by halogen;
R₁₀ each independently represents hydrogen, alkyl, haloalkyl, phenyl, or phenyl substituted by at least one group selected from halogen, cyano, nitro, alkyl, haloalkyl, alkoxycarbonyl, alkylthio, alkylsulfonyl, alkoxy, and haloalkoxy.

2. The benzene ring-substituted pyrimidinecarboxylic acid compound according to claim 1, wherein it is **characterized in that**,
X represent unsubstituted or halogen-substituted C1-C8 alkyl;
Z represents bromine, halo C1-C8 alkyl, or C3-C8 cycloalkyl;
R₅ represents unsubstituted or halogen-substituted C1-C8 alkyl;
W represents hydrogen or its metal salt, amine salt, C1-C8 alkyl, C2-C8 alkenyl, C2-C8 alkynyl, C3-C8 cycloalkyl, C3-C8 cycloalkenyl, heterocyclyl, aryl, 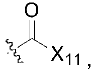 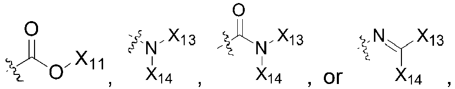 wherein, the "C1-C8 alkyl", "C2-C8 alkenyl" or "C2-C8 alkynyl" is optionally substituted by at least one group selected from halogen, cyano, nitro, C3-C8 cycloalkyl, tri C1-C8 alkylsilyl, C3-C8 cycloalkenyl, heterocyclyl, aryl, 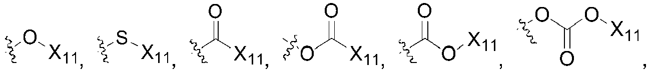 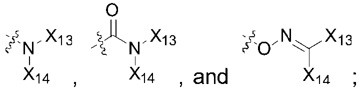 the "C3-C8 cycloalkyl", "C3-C8 cycloalkenyl", "heterocyclyl" or "aryl" is optionally substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₀, -SR₁₀, -(CO)OR₁₀, -(SO₂)R₁₀, - N(R₁₀)₂ and -O-(C1-C8 alkylene)-(CO)OR₁₀, or two adjacent carbon atoms on the ring form a fused ring with -OCH₂CH₂- or -OCH₂O- that is unsubstituted or substituted by halogen;
X₁₁ each independently represents hydrogen, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl, or aryl C1-C8 alkyl, wherein, the "C1-C8 alkyl", "C2-C8 alkenyl" or "C2-C8 alkynyl" is optionally substituted by at least one group selected from halogen and C1-C8 alkoxy; the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" or "aryl C1-C8 alkyl" is optionally substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₀, -SR₁₀, -(CO)OR₁₀, -(SO₂)R₁₀, -N(R₁₀)₂ and -O-(C1-C8 alkylene)-(CO)OR₁₆, or two adjacent carbon atoms on the ring form a fused ring with -OCH₂CH₂- or -OCH₂O- that is unsubstituted or substituted by halogen;
X₁₃ and X₁₄ each independently represent hydrogen, halogen, cyano, C1-C8 alkoxy, C1-C8 alkoxy C1-C8 alkyl, C1-C8 alkylcarbonyl, C1-C8 alkoxycarbonyl, C1-C8 alkylsulfonyl, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, aryl, aryl C1-C8 alkyl, heterocyclyl or heterocyclyl C1-C8 alkyl, or the CX₁₃X₁₄ group together forms a 5-8 membered carbocycle or oxygen-, sulfur- or nitrogen-containing heterocycle, or the NX₁₃X₁₄ group together forms 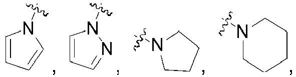 or 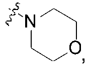 wherein, the "C1-C8 alkyl", "C2-C8 alkenyl" or "C2-C8 alkynyl" is optionally substituted by halogen; the "C3-C8 cycloalkyl", "C3-C8 cycloalkyl C1-C8 alkyl", "C3-C8 cycloalkenyl", "C3-C8 cycloalkenyl C1-C8 alkyl", "aryl", "aryl C1-C8 alkyl", "heterocyclyl" or "heterocyclyl C1-C8 alkyl" is optionally substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₀, -SR₁₀, - (CO)OR₁₀, -(SO₂)R₁₀, -N(R₁₀)₂ and -O-(C1-C8 alkylene)-(CO)OR₁₆, or two adjacent carbon atoms on the ring form a fused ring with -OCH₂CH₂- or -OCH₂O- that is unsubstituted or substituted by halogen; the "5-8 membered carbocycle or oxygen-, sulfur-, or nitrogen-containing heterocycle" is optionally substituted by at least one group selected from C1-C8 alkyl, C1-C8 alkoxycarbonyl and benzyl, or forms a fused ring structure with aryl or heterocyclyl; the 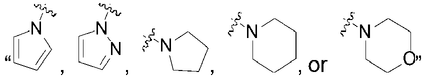 is optionally substituted by at least one group selected from oxo, C1-C8 alkyl, and C1-C8 alkoxycarbonyl;
R₁₀ each independently represents hydrogen, C1-C8 alkyl, halo C1-C8 alkyl, phenyl, or phenyl substituted by at least one group selected from halogen, cyano, nitro, C1-C8 alkyl, halo C1-C8 alkyl, C1-C8 alkoxycarbonyl, C1-C8 alkylthio, C1-C8 alkylsulfonyl, C1-C8 alkoxy, and halo C1-C8 alkoxy.

3. The benzene ring-substituted pyrimidinecarboxylic acid compound according to claim 1 or 2, wherein it is **characterized in that**,
X represents unsubstituted or halogen-substituted C1-C6 alkyl;
Z represents bromine, halo C1-C6 alkyl, or C3-C8 cycloalkyl;
R₅ represents unsubstituted or halogen-substituted C1-C6 alkyl;
W represents hydrogen or its sodium salt, potassium salt, C1-C6 alkyl, C2-C6 alkenyl, C2-C6 alkynyl, C3-C6 cycloalkyl, C3-C6 cycloalkenyl, heterocyclyl, aryl, 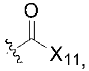 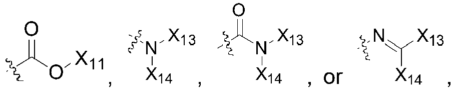 wherein, the "C1-C6 alkyl", "C2-C6 alkenyl" or "C2-C6 alkynyl" is optionally substituted by 1, 2 or 3 groups selected from halogen, cyano, nitro, C3-C6 cycloalkyl, tri C1-C6 alkylsilyl, C3-C6 cycloalkenyl, heterocyclyl, aryl, 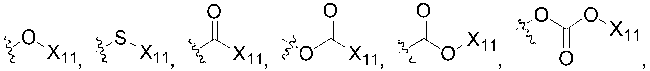 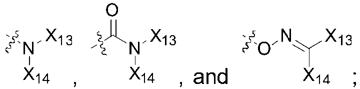 the "C3-C6 cycloalkyl", "C3-C6 cycloalkenyl", "heterocyclyl" or "aryl" is optionally substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₀, -SR₁₀, -(CO)OR₁₀, -(SO₂)R₁₀, - N(R₁₀)₂ and -O-(C1-C6 alkylene)-(CO)OR₁₀, or two adjacent carbon atoms on the ring form a fused ring with -OCH₂CH₂- or -OCH₂O- that is unsubstituted or substituted by halogen;
X₁₁ each independently represents hydrogen, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl, or aryl C1-C6 alkyl, wherein, the "C1-C6 alkyl", "C2-C6 alkenyl" or "C2-C6 alkynyl" is optionally substituted by at least one group selected from halogen and C1-C6 alkoxy; the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" or "aryl C1-C6 alkyl" is optionally substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₀, -SR₁₀, -(CO)OR₁₀, -(SO₂)R₁₀, -N(R₁₀)₂ and -O-(C1-C6 alkylene)-(CO)OR₁₆, or two adjacent carbon atoms on the ring form a fused ring with -OCH₂CH₂- or -OCH₂O- that is unsubstituted or substituted by halogen;
X₁₃ and X₁₄ each independently represent hydrogen, halogen, cyano, C1-C6 alkoxy, C1-C6 alkoxy C1-C6 alkyl, C1-C6 alkylcarbonyl, C1-C6 alkoxycarbonyl, C1-C6 alkylsulfonyl, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, aryl, aryl C1-C6 alkyl, heterocyclyl or heterocyclyl C1-C6 alkyl, or the CX₁₃X₁₄ group together forms a 5-8 membered saturated carbocycle, 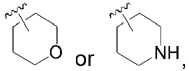 or the NX₁₃X₁₄ group together forms 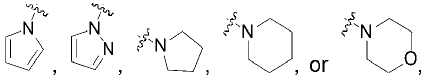 wherein, the "C1-C6 alkyl", "C2-C6 alkenyl" or "C2-C6 alkynyl" is optionally substituted by halogen; the "C3-C6 cycloalkyl", "C3-C6 cycloalkyl C1-C6 alkyl", "C3-C6 cycloalkenyl", "C3-C6 cycloalkenyl C1-C6 alkyl", "aryl", "aryl C1-C6 alkyl", "heterocyclyl" or "heterocyclyl C1-C6 alkyl" is optionally substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₀, -SR₁₀, - (CO)OR₁₀, -(SO₂)R₁₀, -N(R₁₀)₂ and -O-(C1-C6 alkylene)-(CO)OR₁₆, or two adjacent carbon atoms on the ring form a fused ring with -OCH₂CH₂- or -OCH₂O- that is unsubstituted or substituted by halogen; the "5-8 membered saturated carbocycle, 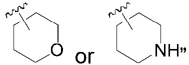 is optionally substituted by 1, 2 or 3 groups selected from C1-C6 alkyl, C1-C6 alkoxycarbonyl and benzyl, or forms a fused ring structure with aryl or heterocyclyl; the 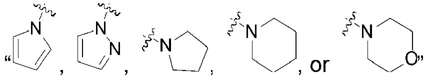 is optionally substituted by 1, 2 or 3 groups selected from oxo, C1-C6 alkyl, and C1-C6 alkoxycarbonyl;
R₁₀ each independently represents hydrogen, C1-C6 alkyl, halo C1-C6 alkyl, phenyl, or phenyl substituted by 1, 2 or 3 groups selected from halogen, cyano, nitro, C1-C6 alkyl, halo C1-C6 alkyl, C1-C6 alkoxycarbonyl, C1-C6 alkylthio, C1-C6 alkylsulfonyl, C1-C6 alkoxy, and halo C1-C6 alkoxy.

4. The benzene ring-substituted pyrimidinecarboxylic acid compound according to any one of claims 1 to 3, wherein it is **characterized in that**,
X represents methyl, CHF₂, CH₂F, or CF₃;
Z represents bromine, CF₃, or cyclopropyl;
R₃ represents fluorine, chlorine, or amino;
R₄ represents hydrogen or fluorine;
R₅ represents methyl, CHF₂, CH₂F, or CF₃;
preferably, the compound is selected from any one in Table 1 in the Specification.

5. An intermediate compound, as shown in the general formula II: 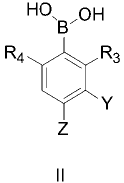 wherein, the definitions of substituents R₃, R₄, Y and Z are as described according to any one of claims 1 to 4.

6. A method for preparing the benzene ring-substituted pyrimidinecarboxylic acid compound according to any one of claims 1 to 5, wherein it is **characterized in that**, it comprises the following steps:
subjecting the compound shown in the general formula II and the compound shown in the general formula III to reaction to produce the compound as shown in the general formula I, and the reaction equation is as follows: 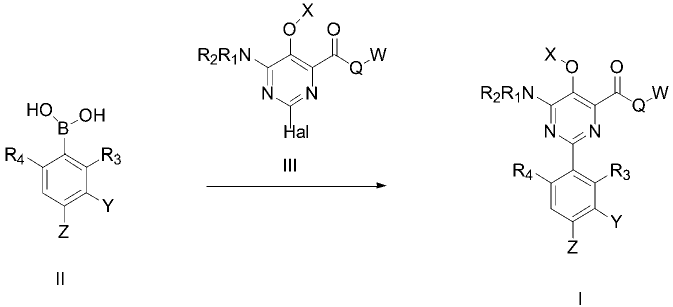
wherein, Hal represents halogen, preferably Cl; the definitions of substituents R₁, R₂, R₃, R₄, X, Y, Z, Q and W are as described according to any one of claims 1 to 4.

7. The method according to claim 6, wherein it is **characterized in that**, the reaction is carried out in the presence of a catalyst, a base, and a solvent; preferably, the catalyst is a palladium catalyst [e.g., Pd(dppf)Cl₂·CH₂Cl₂, Pd(dppf)Cl₂, Pd(PPh₃)₄, methanesulfonato(2-dicyclohexylphosphino-2',6'-dimethoxybiphenyl)(2'-methylamino-1,1'-biphenyl-2-yl)palladium(II), or methanesulfonato(tri-tert-butylphosphino)(2'-methylamino-1,1'-biphenyl-2-yl)palladium(II), etc.] or nickel catalyst; preferably, the base is selected from at least one of inorganic bases (e.g., K₂CO₃, Na₂CO₃, Cs₂CO₃, NaHCO₃, KHCO₃, KF, CsF, K₃PO₄, K₂HPO₄, NaOH, KOH, etc.) and organic bases (e.g., pyrazole, triethylamine, DIEA, potassium trimethylsilanolate, AcOK, AcONa, MeONa, EtONa, *t*-BuONa, etc.); preferably, the solvent is organic solvent/water, the volume ratio of which is preferably 20/1-1/1, and the organic solvent is preferably dioxane, toluene, DMF, DMSO, DMA, methanol, ethanol, isopropanol, *n*-butanol, acetonitrile, dichloroethane, dichloromethane, or ethyl acetate.

8. A herbicidal composition, wherein it is **characterized in that**, it comprises a herbicidally effective amount of at least one of the benzene ring-substituted pyrimidinecarboxylic acid compounds according to any one of claims 1 to 4; preferably, also comprising a formulation auxiliary; more preferably, also comprising an additional active substance selected from one or more of the following compounds:
(1) HPPD inhibitors: 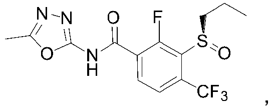 bipyrazone, fenpyrazone, cypyrafluone, tripyrasulfone;
(2) ALS inhibitors: florasulam;
(3) Synthetic hormones: halauxifen-methyl, florpyrauxifen-benzyl, fluroxypyr-meptyl, benazolin-ethyl, clopyralid, aminopyralid, picloram, MCPA-isooctyl;
(4) PSII inhibitors: propanil, bentazone;
(5) DOXP inhibitors: 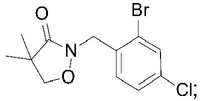
(6) FAT inhibitors: 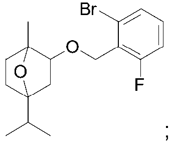
further preferably, the weight ratio of the benzene ring-substituted pyrimidinecarboxylic acid compound according to any one of claims 1 to 4 and the aforementioned active substance in the composition is 1:500-20:1, 1:400-10:1, 1:300-5:1, 1:200-2:1, 1:100-2:1, 1:50-2:1, 1:30-2:1, 1:20-2:1, 1:10-2:1, or 1:5-1:1.

9. A method for controlling weeds, wherein it is **characterized in that**, it comprises applying a herbicidally effective amount of at least one of the benzene ring-substituted pyrimidinecarboxylic acid compounds according to any one of claims 1 to 4 or the herbicidal composition according to claim 8 to a plant or a weedy area.

10. Use of at least one of the benzene ring-substituted pyrimidinecarboxylic acid compounds according to claims 1 to 4 or the herbicidal composition according to claim 8 in controlling weeds; preferably, the benzene ring-substituted pyrimidinecarboxylic acid compound is used for preventing and controlling a weed among a useful crop, and the useful crop is a transgenic crop or a crop treated by a genome editing technique.
